# EUROPEAN PATENT SPECIFICATION

(11) **EP 4 008 238 B1**
(45) Date of publication and mention of the grant of the patent: **03.12.2025**
(21) Application number: 21211873.1
(22) Date of filing: 02.12.2021
(51) Int. Cl.: A61B 3/028, A61B 3/06, A61B 3/024

(54) **OPTOMETRY CONTROL PROGRAM AND SUBJECTIVE OPTOMETRY SYSTEM**
OPTOMETRIESTEUERUNGSPROGRAMM UND SUBJEKTIVES OPTOMETRIESYSTEM
PROGRAMME DE CONTRÔLE D'OPTOMÉTRIE ET SYSTÈME D'OPTOMÉTRIE SUBJECTIVE

(30) Priority: 04.12.2020 JP 2020201490
(43) Date of publication of application: 08.06.2022
(73) Proprietor: NIDEK CO., LTD., Gamagori Aichi (JP)
(72) Inventor: SHIMAZAKI, Arisa, Gamagori, Aichi (JP); TERABE, Hirohisa, Gamagori, Aichi (JP); HORINO, Taeko, Gamagori, Aichi (JP)
(74) Representative: Hoefer & Partner Patentanwälte mbB

(56) References cited:
- EP-A1- 3 381 350
- EP-A1- 3 607 873
- WO-A1-2020/172203
- WO-A1-2021/200605
- US-A- 6 048 064
- US-A1- 2019 099 073

## Description

### TECHNICAL FIELD

The present disclosure relates to an optometry control program executed in a subjective optometry system, and the subjective optometry system.

### BACKGROUND ART

A subjective optometry device that measures optical characteristics such as an eye refractive power of a subject eye by arranging optical elements in front of an examinee and presenting an examination target on the subject eye through the optical elements, is known. For example, a subjective optometry device disclosed in JP-A-2020-018712 includes an eye refractive power measurement unit, a target presentation unit, and a controller. The eye refractive power measurement unit switches the optical elements to be arranged in an optometry window among a plurality of optical elements provided by the calibration optical system using a drive portion. The target presentation unit switches an examination target to be presented to the subject eye. The controller detects a user's operation on an operation panel and transmits a drive signal to the eye refractive power measurement unit and the target presentation unit based on the detected operation.

In order to smoothly carry out the subjective optometry while reducing the burden on an examiner by using such a subjective optometry device, a self-optometry in which the examinee him/herself carry out an examination has been studied. When a problem occurs in a proceeding of the self-optometry, it is necessary for an operator such as the examiner to assist the proceeding of the self-optometry. However, while the examinee carries out the self-optometry, it was difficult for the operator to grasp a situation of the self-optometry and to easily start an assistance-required examination.
US 2019/099073 A1 discloses a subjective optometry apparatus which includes a subjective measurement portion which subjectively measures optical characteristics of an subject eye, an objective measurement portion which objectively measures optical characteristics of the subject eye, a control portion which causes the objective measurement portion to objectively measure the optical characteristics during the measurement by the subjective measurement portion, and a display control portion which performs a control to display an eye diagram representing the subject eye and an imaging position of a target light flux incident on the subject eye. The display control portion performs a control to display the imaging position based on the objectively measured optical characteristics.
EP 3 381 350 A1 discloses a subjective optometry apparatus which includes a light projecting optical system that projects a target light flux to an examinee's eye, a calibration optical system that is disposed in an optical path from the light projecting optical system to the examinee's eye, and changes optical characteristics of the target light flux, an optical element that guides the target light flux of which the optical characteristics is changed by the calibration optical system to the examinee's eye, and a correction portion that corrects distortion of the target light flux caused by the target light flux passing through an optical path deviated from an optical axis of the optical element. The target light flux passes through the optical path deviated from the optical axis of the optical element to be guided to the examinee's eye. The subjective optometry apparatus subjectively measures optical characteristics of the examinee's eye using the target light flux guided to the examinee's eye.
EP 3 607 873 A1 discloses a subjective optometry apparatus which subjectively measures optical characteristics of a subject eye. The subjective optometry apparatus includes a light projecting optical system, an optical path switching portion that switches between a first optical path having a first examination distance and a second optical path having a second examination distance, and an examination distance changing portion that optically changes the first examination distance or the second examination distance. Based on examination distance information, the examination distance changing unit causes the optical path switching portion to set an optical path to either the first optical path or the second optical path, and drives a distance changing optical member in the set optical path to optically change the examination distance to an examination distance different from the first examination distance and the second examination distance.

### SUMMARY OF INVENTION

A technical object of the present disclosure is to provide an optometry control program and a subjective optometry system that can grasp a situation of a self-optometry and easily start an assistance-required examination. This object is solved by the features of the independent claims. The dependent claims contain advantageous embodiments of the present invention.
(1) An optometry control program executed by a first information processing device in a subjective optometry system that includes a subjective optometry device which is used for subjectively measuring optical characteristics of a subject eye and which includes a calibration optical system that changes optical characteristics of a target light flux presented to the subject eye and a target presentation portion that presents a target to the subject eye and that includes a dedicated operation portion suitable for inputting a response of the subjective optometry by an examinee, and the first information processing device connected to the subjective optometry device, the optometry control program including:
   a self-optometry program that automatically proceeds with an optometry according to the response input by the examinee via the operation portion,
   in which the optometry control program includes instructions which, when the self-optometry program is executed by a control portion of the first information processing device, cause the first information processing device to perform:
      a self-optometry proceeding step of controlling the calibration optical system and the target presentation portion to perform a plurality of examination items based on at least a procedure of a self-optometry that is automatically proceeded; and
      a self-optometry assistance step of performing an assistance action to assist a proceeding of the self-optometry in a case where a problem occurs in the proceeding of the self-optometry, and
   the self-optometry assistance step includes:
      a display control step of displaying an optometry flow, on a display device, in which the plurality of examination items are arranged in order of the procedure of the self-optometry;
      a selection receipt step of receiving an operator's input of a selection instruction to select a predetermined examination item in the optometry flow displayed on the display device; and
      a selection performing step of performing an examination corresponding to the predetermined examination item based on the received input of the selection instruction.
(2) In the optometry control program according to the above-described (1),
   the self-optometry assistance step includes:
      a call receipt step of receiving an input of a call performing instruction to perform a call action for calling an examiner who is the operator; and
      a call performing step of performing the call action according to the call performing instruction received in the call receipt step, and
   the control portion of the first information processing device restricts the input of the selection instruction from an operation portion through which the examiner operates the subjective optometry device until the call performing instruction is input, and releases the restriction on the input from the operation portion in a case where the call performing instruction is input.
(3) In the optometry control program according to the above-described (1) or (2),
   in the display control step, the examination item is displayed on the display device as the optometry flow, the displayed examination item being limited to a predetermined examination item including at least an examination item being performed.
(4) In the optometry control program according to the above-described (3),
   in the display control step, the predetermined examination item is displayed on the display device as the optometry flow, the displayed predetermined examination item being limited to the predetermined examination item further including at least one of an examination item performed after the examination item being performed and an examination item performed before the examination item being performed.
(5) In the optometry control program according to the above-described (3) or (4),
   in the display control step, when the examination item is displayed, the displayed examination item being limited to the predetermined examination item, the limitation on displaying the examination item as the optometry flow is released in a case where the call performing instruction is input.
(6) In the optometry control program according to any one of the above-described (1) to (5),
   in the display control step, an identification by which at least an examination item being performed enables to be identified is displayed in the optometry flow.
(7) In the optometry control program according to any one of the above-described (1) to (6),
   in the self-optometry assistance step, at least one of the procedure of the self-optometry and the plurality of examination items is variable according to an instruction input by the operator, and
   in the display control step, the optometry flow is displayed on the display device while being changed based on at least one of the procedure of the self-optometry and the plurality of examination items changed according to the instruction input by the operator.
(8) In the optometry control program according to any one of the above-described (1) to (7),
   in the selection performing step, the self-optometry is restarted from an examination corresponding to the selected predetermined examination item in the optometry flow based on the input of the selection instruction.
(9) **In** the optometry control program according to any one of the above-described (1) to (8),
   the self-optometry assistance step is performed according to an instruction input to a second information processing device, which is another information processing device connected to the first information processing device via a network.
(10) A subjective optometry system including:
   a subjective optometry device which is used for subjectively measuring optical characteristics of a subject eye and which includes a calibration optical system that changes optical characteristics of a target light flux presented to the subject eye and a target presentation portion that presents a target to the subject eye and which includes a dedicated operation portion suitable for inputting a response of the subjective optometry by an examinee; and
   a first information processing device connected to the subjective optometry device,
   in which the first information processing device is configured to execute a self-optometry program that automatically proceeds with an optometry according to the response input by the examinee via the operation portion,
   the self-optometry program includes instructions which, when the self-optometry program is executed by the first information processing device, cause the first information processing device to perform:
      a self-optometry proceeding step of controlling the calibration optical system and the target presentation portion to perform a plurality of examination items based on at least a procedure of a self-optometry that is automatically proceeded; and
      a self-optometry assistance step of performing an assistance action to assist a proceeding of the self-optometry in a case where a problem occurs in the proceeding of the self-optometry, and
   the self-optometry assistance step includes:
      a display control step of displaying an optometry flow, on a display device, in which the plurality of examination items are arranged in order of the procedure of the self-optometry;
      a selection receipt step of receiving an operator's input of a selection instruction to select a predetermined examination item in the optometry flow displayed on the display device; and
      a selection performing step of performing an examination corresponding to the predetermined examination item based on the received input of the selection instruction.

### BRIEF DESCRIPTION OF DRAWINGS

FIG. 1 is a block diagram showing a schematic configuration of a subjective optometry system 100.
FIG. 2 is a flowchart of optometry control processing performed by a first information processing device 2A.
FIG. 3 is a diagram showing an example of a self-optometry assistance screen used in the present embodiment.
FIGs. 4A and 4B in combination are flowcharts of self-optometry assistance processing performed during the optometry control processing.
FIG. 5 is a diagram showing an example of a display screen displaying an optometry flow.

### DESCRIPTION OF EMBODIMENTS

### <Overview>

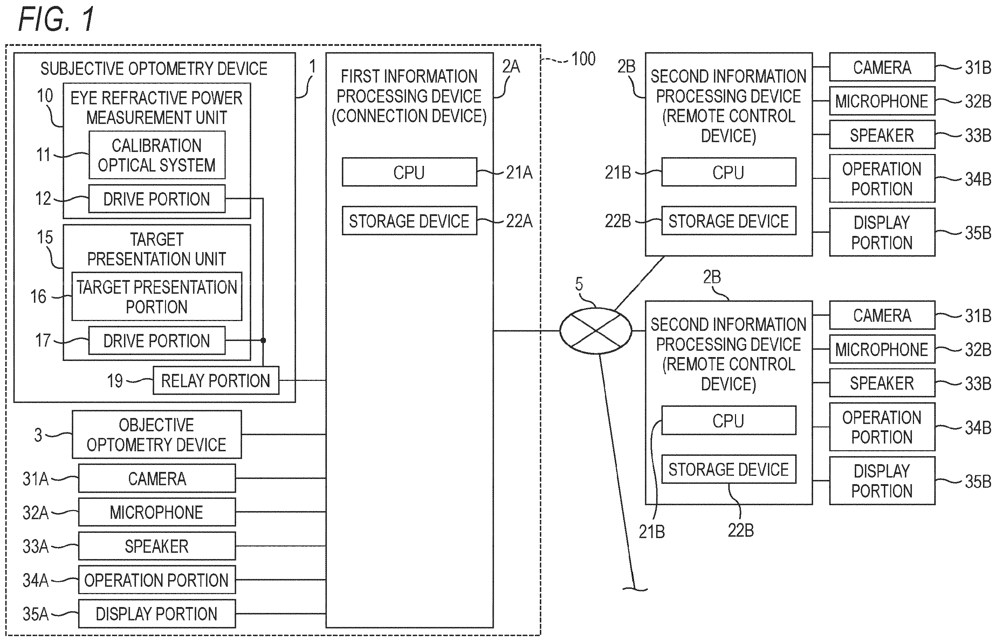

Hereinafter, one of typical embodiments will be described with reference to the drawings. FIG. 1 to FIG. 5 are diagrams for explaining the subjective optometry system according to the present embodiment. Items classified by <> below can be used independently or in relation to each other.

The subjective optometry system (for example, a subjective optometry system 100) exemplified in the present disclosure includes a subjective optometry device (for example, a subjective optometry device 1) and a first information processing device (a first information processing device 2A). The subjective optometry device includes a calibration optical system (a calibration optical system 11) that changes optical characteristics of a target light flux presented to a subject eye and a target presentation portion (a target presentation portion 16) that presents a target to the subject eye, and is used to subjectively measure the optical characteristics of the subject eye. The first information processing device is an information processing device that is connected to the subjective optometry device (hereinafter, the first information processing device may be referred to as a "connection device"). The optometry control program in the present disclosure includes a self-optometry program. The self-optometry program is a program for automatically proceeding with an optometry according to a response input by an examinee.

For example, in the present embodiment, when the self-optometry program is executed by a control portion of the first information processing device, a self-optometry proceeding step, a response acquisition step, a calibration value storage step, and a self-optometry assistance step are performed by the first information processing device. In the self-optometry proceeding step, the first information processing device outputs a plurality of presentation instruction signals, for instructing to present the target to the examinee, to the subjective optometry device based on at least in order of a procedure of the automatically proceeded self-optometry (that is, along the procedure). In the self-optometry proceeding step, the first information processing device may control the calibration optical system and the target presentation portion to perform a plurality of examination items based on the procedure of the automatically proceeded self-optometry. In the response acquisition step, the first information processing device acquires the response input by the examinee who visually recognizes a presented target. In the calibration value storage step, the first information processing device stores a calibration value of the optical characteristics of the subject eye that is acquired based on the response acquired in the response acquisition step and the optical characteristics of the target and the target light flux presented by the subjective optometry device at the time of acquiring the response. In the self-optometry assistance step, when there is a problem in proceeding with the self-optometry, the first information processing device performs an assistance action to assist the proceeding of the self-optometry.

According to the subjective optometry system in the present disclosure, by executing the self-optometry program, the optometry for the examinee can be appropriately carried out without the examiner proceeding with the optometry. Furthermore, if there is a problem in the proceeding of the self-optometry, an assistance action is performed to assist the proceeding of the self-optometry. That is, a function to assist the proceed of the self-optometry is incorporated into the function of the self-optometry program. Therefore, for example, while the self-optometry program is executed, at least one of the self-optometry assistance actions based on an advice from the examiner is performed. Therefore, the subjective optometry is appropriately carried out while the burden on the examiner is reduced.

Various devices can be used as the first information processing device that executes the optometry control program. For example, a personal computer (hereinafter, referred to as a "PC") may be used as the first information processing device. A server, a mobile terminal, a smartphone, or the like may be used as the first information processing device.

The first information processing device may be configured by combining a plurality of devices. For example, the first information processing device may be configured with a device such as a personal computer and a dedicated controller including a controller and a storage device. The dedicated controller itself may be provided with a function as a device such as a personal computer including a CPU, and may be configured as a first information processing device.

A storage device that stores the optometry control program can be selected as appropriate. For example, the optometry control program may be stored in a storage device built in the first information processing device, or may be stored in a storage device that is attachable to and detachable from the first information processing device. The optometry control program may be stored in a storage device built in the dedicated controller described above. In addition, the optometry control program may be stored in a plurality of storage devices.

In the self-optometry assistance step, when the status of the self-optometry being carried out meets a predetermined condition or when the instruction to perform the assistance action is input, the assistance action to assist the proceeding of self-optometry may be performed. In this case, when a situation in which the self-optometry cannot be carried out on the examinee occurs, the self-optometry assistance action is performed appropriately.

The condition for the situation of self-optometry when performing the assistance action can be appropriately selected. For example, when a predetermined time elapses without the response of the examinee being input, the control portion may determine that the condition for performing the assistance action is satisfied. In addition, when the response input by the examinee is inappropriate, the control portion may also determine that the condition for performing the assistance action is satisfied. The method for determining whether or not the response is inappropriate can be appropriately selected. For example, when the input response does not meet a response condition set in advance (for example, when a response different from the response candidate requested for the examinee is input, the same responses are input consecutively more than a predetermined number of times, when the responses more than the number of responses requested for the examinee are input, or the like), the control portion may determine that the input response is inappropriate.

The assistance action performed in the self-optometry assistance step may include a calling action for calling the examiner. In this case, since the examiner is called when there is a problem with the proceeding of the self-optometry, subsequent optometry is carried out appropriately.

The self-optometry assistance step may include a manual proceeding step of outputting a presentation instruction signal, to the subjective optometry device, for proceeding with at least a part of the procedure of the self-optometry according to the instruction input by the examiner. **In** this case, the examiner can manually proceed with problematic examination and the like among the procedures that were planned to proceed by self-optometry. Therefore, the optometry for the examinee is assisted more appropriately.

The self-optometry assistance step may include a calibration value revision step of revising at least one or a plurality of calibration values stored by the self-optometry according to the instructions input by the examiner. **In** this case, for example, even if there is a calibration value of which the measurement by the self-optometry fails, the calibration value is revised by decision of the examiner. Therefore, the optometry for examinee proceeds smoothly.

The self-optometry assistance step may include a test omission step of omitting at least a part of the procedure according to instruction input by the examiner. In this case, the examiner can omit a procedure, among the predetermined procedures, that is determined to be unnecessary. Therefore, the optometry for examinee proceeds smoothly.

The method for inputting the instructions by the examiner for omitting at least a part of the procedures can be appropriately selected. For example, the examiner may input an instruction, to the first information processing device, to select tests to be omitted among a plurality of tests performed along the procedure. In this case, the control portion of the first information processing device may omit the selected test from the procedure. The examiner may input instruction to set a timing for restarting temporarily stopped self-optometry to a timing after the omitted procedure, to the first information processing device. **In** this case, the control portion of the first information processing device may omit some procedures by restarting the self-optometry from the timing set.

The self-optometry assistance step may include a proxy response acquisition step. **In** the proxy response acquisition step, when the target is presented to the examinee according to the procedure by the self-optometry proceeding step, the control portion acquires the response of the examinee who visually recognizes the presented target according to the instruction input by the examiner. In this case, the examiner can hear the response of the examinee who visually recognizes the target and input the heard response by him/herself, in the process of presenting the target to the examinee in an order along the procedure of the self-optometry. Therefore, even if the examinee does not grasp the method of response and the like in the self-optometry, the examiner can appropriately assist the self-optometry by inputting the response heard from the examinee.

The self-optometry assistance step may include a continuous restart step of restarting the temporarily stopped self-optometry from next procedure of procedure which has already been completed (for example, completed test and the like) among the procedures. **In** this case, the self-optometry is restarted from next procedure of procedure which has already been completed among the procedures. Therefore, even after the assistance for the self-optometry is completed, the self-optometry is appropriately continued along the procedure.

The self-optometry assistance step may include a designed restart step of restarting the temporarily stopped self-optometry from procedure designated by the examiner (for example, the designated test or the like) among the procedures. **In** this case, when the problem that occurred during the self-optometry is solved, the examiner can restart the self-optometry from the procedure designed by the examiner. Therefore, the excessive increase of burden on the examiner to assist the optometry is reduced.

The procedure of the self-optometry may define a plurality of tests (examination) performed for the examinee and a performing order of the plurality of tests. **In** the self-optometry proceeding step, a plurality of tests may be performed in order according to the procedure. **In** this case, a plurality of tests for the examinee is proceeded appropriately and automatically. Furthermore, the examiner can appropriately assist the test judged that the assistance is necessary (for example, the test for which a good calibration value is not acquired by the self-optometry) among the plurality of tests.

The self-optometry assistance step may include a procedure display step of displaying the procedure on a display device. The display device may be disposed at a position where the examiner waits. That is, the display device may be disposed at a position different from the position where the subjective optometry device is disposed, or may be disposed at a position same as the position where the subjective optometry device is disposed. **In** the designated restart step, on the procedure displayed on the display device, the input of the instruction to designate the procedure for restarting the self-optometry (hereinafter, referred to as "restarting procedure") may be received. **In** this case, the examiner can designate the restarting procedure for restarting the self-optometry in a state where details and situation of the procedure is appropriately grasped by the displayed procedure.

Specific methods for receiving the input of the restarting procedure on the displayed procedure may be appropriately selected. For example, a touch panel may be provided in a display area of the display device. **In** this case, the control portion may process the procedure (for example, specific test) corresponding to the position where the touch panel is operated among the displayed procedures as the restarting procedure designed by the examiner. **In** addition, a mouse or the like for moving the pointer within the display area in the display device may be provided. **In** this case, the control portion may process the procedure corresponding to the position of the pointer among the displayed procedures as the restarting procedure designed by the examiner.

Among the entire procedures (for example, a plurality of tests or the like included in the procedure), the control portion may display at least one of the procedure being performed at that time, the procedure being performed when a problem occurs in the proceeding of the procedure, and the like. In this case, the examiner can appropriately grasp the proceeding of the self-optometry by the displayed procedure.

As described above, various advantages occur by restarting the temporarily stopped self-optometry. For example, in a cross cylinder-test, which is one of the tests performed by the subjective optometry system, two targets (for example, point-cloud target) are presented to the examinee. However, when a relative position between the examination window of the calibration optical system and the subject eye is deviated, the examinee may be able to see only one of the two point-cloud targets (point-cloud). **In** this case, the examinee cannot carry out the self-optometry. However, according to the subjective optometry system according to the present disclosure, the examiner advises to adjust the position of the subject eye with respect to the examination window while the self-optometry is temporarily stopped, and assists the proceeding of the cross cylinder-test. Then, the self-optometry which is independently carried out by the examinee can be restarted. As described above, when the temporarily stopped self-optometry is restarted, the effects useful for both the examiner and the examinee can be obtained.

The self-optometry assistance step may be performed according to the instructions input to a second information processing device which is another information processing device connected to the first information processing device via a network. **In** this case, even if the examiner is in a remote location different from the place of examinee, the examiner can appropriately assist the self-optometry carried out by the examinee from the remote location by inputting the instruction to the second information processing device.

When there is a problem in the proceeding of the self-optometry (for example, when the instruction to perform the assistance action is input, and the like), the control portion of the first information processing device may output an instruction, to the second information processing device, to perform the calling action for calling the examiner. In this case, even when the examiner is in a remote location different from the place of examinee, the examiner can easily grasp that there is a problem in the proceeding of the self-optometry.

The self-optometry assistance step may be performed according to an instruction directly input to the first information processing device (for example, an instruction input to the operation portion of the first information processing device). That is, the examiner at the place where the subjective optometry device and the first information processing device are disposed may input the instruction to perform the self-optometry assistance step. In this case, for example, an operation portion of the first information processing device used for inputting the instruction from the examiner may be a dedicated controller provided in the subjective optometry device, or may be a general-purpose user interface such as a mouse, a tablet, or the like.

In addition, when the second information processing device is used, the number of second information processing devices may be one, or two or more. In addition, similarly to the first information processing device, various devices (for example, a PC, a mobile terminal, a smartphone, and the like) can be used as the second information processing device. The second information processing device may have been connected to the first information processing device already when the self-optometry is carried out, or may be connected to the first information processing device after a problem occurs in the self-optometry.

When the second information processing device is used, each control portion of the first information processing device and the second information processing device may perform a remote conversation step of assisting a conversation between the examiner and the examinee by transmitting and receiving at least audio data (which may be both the audio data and image data). In this case, the examiner at the remote location can appropriately assist the self-optometry by grasping situation of the examinee through the conversation.

### <Optometry Flowchart Display>

As a self-optometry assistance step, an optometry flow (optometry flowchart) (for example, optometry flow 60) may be used to assist the self-optometry. Hereinafter, a configuration using the optometry flow will be described.

In the present embodiment, when the self-optometry program is executed by the control portion of the first information processing device, the self-optometry proceeding step and the self-optometry assistance step may be performed by the first information processing device. For example, in the self-optometry proceeding step, the first information processing device controls the calibration optical system and the target presentation section to perform a plurality of examination items based on at least the automatically proceeded procedure of the self-optometry (that is, along the procedure). For example, in the self-optometry assistance step, the first information processing device performs the assistance action to assist the proceeding of the self-optometry if there is a problem in the proceeding of the self-optometry.

**In** addition, for example, the self-optometry assistance step may include a display control step, a selection receipt step, and a selection performing step. For example, in the display control step, an optometry flow in which a plurality of examination items is arranged in order of the procedure of the self-optometry is displayed on the display device. For example, in the selection receipt step, the input of the selection instruction to select the predetermined examination items in the optometry flow displayed on the display device is received from the operator (for example, at least one of the examiner and the examinee). **In** addition, for example, in the selection performing step, the examination corresponding to the predetermined examination item is performed based on the input of the selection instruction received in the selection receipt step.

With such a configuration, for example, by checking the optometry flow displayed on the display device, it is possible to grasp the situation of the self-optometry such as the procedure and the situation of proceeding, and to select the examination item that the operator wants to assist. Therefore, for example, the operator can select the examination item to be assisted while appropriately grasping the situation of self-optometry such as the details of the procedure and the situation of proceeding by the displayed optometry flow, and can easily start the assistance-required examination. In addition, for example, at the time of self-optometry, even if the operator does not stay close to the examinee all the time and does not grasp the situation of the self-optometry, it is possible to easily start the examination that requires the assistance.

For example, the configuration using the optometry flow in the present embodiment can be used in various configurations. For example, during the self-optometry, to perform the assistance of the self-optometry, a predetermined examination item may be selected in the optometry flow, and the assistance for the examination corresponding to the selected examination item may be performed.

For example, when restarting the self-optometry, a predetermined examination item may be selected in the optometry flow, and the self-optometry is restarted from the selected examination item. In this case, for example, in the selection performing step, the self-optometry may be restarted from the examination corresponding to the selected predetermined examination item based on the input of the selection instruction for the optometry flow. With such a configuration, it is possible to input the instruction to select the examination item from which the self-optometry is restarted while the operator checks the optometry flow displayed on the display device. Therefore, the operator can designate an examination process from which the self-optometry is restarted in a state where details of the procedure, the situation of proceeding, and the like is appropriately grasped by the displayed optometry flow.

When performing the examination of the selected examination item, any method can be set as a method of setting the start state when starting the examination. For example, when performing the examination of the selected examination item, in the self-optometry assistance step, the subjective optometry device may be controlled so as to be in the examination state at the time of receiving the input of the selection instruction. In this case, for example, the target at the time of receiving the input of the selection instruction may be presented by the target presentation portion, or the calibration value at the time of receiving the input of the selection instruction may be set in the calibration optical system. As a result, since the examination can be started while maintaining the examination state, it is not necessary to set the examination state from the beginning, and the examination can be performed smoothly. **In** this case, by performing both the presentation of the target and setting of the calibration value, the examination state at the time of receiving the input of the selection instruction can be reproduced. However, any one of the presentation of the target and setting of the calibration value may be performed.

For example, when performing the examination of the selected examination item, in the self-optometry assistance step, the subjective optometry device may be controlled so as to be in the initial state (the state in which the initial value is set) of the examination item at the time of receiving the input of the selection instruction. In this case, for example, the target at the time of starting the examination of the examination item at the time of receiving the input of the selection instruction may be presented by the target presentation portion, or the calibration value at the time of starting the examination of the examination item at the time of receiving the input of the selection instruction may be set in the calibration optical system. As a result, for example, since the examination from the initial state of the examination at the time of receiving the input of the selection instruction can be performed. Therefore, when the examination at the time of receiving the input of the selection instruction is inappropriately performed, the examination can be reset, and the examination can be performed from the starting point of the examination. As a result, the examination can be performed smoothly. By performing both the presentation of the target and setting of the calibration value, the initial state of the examination of the examination at the time of receiving the input of the selection instruction can be reproduced. However, any one of the presentation of the target and setting of the calibration value may be performed.

For example, a first control which control the subjective optometry device so as to be in the examination state at the time of receiving the input of the selection instruction, and a second control which control the subjective optometry device so as to be in the initial state in the examination item at the time of receiving the input of the selection instruction may be selectively performed. In this case, in the operation portion, the first control and the second control may be selected by the examiner. As a result, for example, after confirming the situation of the examination, the examiner can select the device settings at the time performing the assistance by the examiner according to the situation of the examination at the time of receiving the input of the selection instruction.

An initial value set as an initial state may be set based on at least one of objective measurement data, previous glass data, and past subjective measurement data. Of course, for example, an initial value different from the above-described data may be used as the initial value. In this case, for example, any value (for example, a default value (for example, 0, and the like)) may be set as the initial value.

For example, the objective measurement data may be data obtained by objectively measuring the optical characteristics of the subject eye. In this case, for example, the objectively measured optical characteristics of the subject eye include eye refractive power (for example, spherical power, astigmatic power, astigmatic axis angle, and the like), polarization characteristics, inter-pupillary distance, and the like. In addition, for example, the previous glass data may be data obtained by measuring the optical characteristics of the glasses worn by the examinee. In this case, for example, the optical characteristics of the glasses include the refractive power (for example, spherical power, astigmatic power, astigmatic axis angle, and the like), polarization characteristics, inter-pupillary distance, and the like. In addition, for example, the past subjective measurement data may be the subjective measurement data which is a result of the past subjective measurement of the examinee who performs the subjective measurement. For example, the subjective measurement data may be data obtained by subjectively measuring the optical characteristics of the subject eye. In this case, for example, the subjectively measured optical characteristics of the subject eye include the eye refractive power (for example, the spherical power, the astigmatic power, the astigmatic axis angle, and the like), the polarization characteristics, the inter-pupillary distance, and the like.

For example, the display device can be disposed at any position. For example, the display device only needs to be disposed at the position where the operator waits. That is, the display device may be disposed at a position different from the position where the subjective optometry device is disposed, or may be disposed at a position same as the position where the subjective optometry device is disposed. For example, as an example, when being disposed at a position different from the position where the subjective optometry device is disposed, a display portion connected to the second information processing device (for example, the display portion 35B) may be used as the display device. **In** addition, for example, when being disposed at a position same as the position where the subjective optometry device is disposed, a display portion (for example, the display portion 35A) connected to the first information processing device may be used as the display device. The configuration used as the display device is not limited to the above-described configuration, and different display portions may be used.

For example, a method of inputting of the selection instruction to select the predetermined examination items, in the optometry flow displayed on the display device, can be appropriately selected. For example, the operator may use the operation portion to input the selection instruction to select the predetermined examination items. For example, the operation portion can be disposed at any position. For example, the operation portion only needs to be disposed at the position where the operator waits. That is, the operation portion may be disposed at a position different from the position where the subjective optometry device is disposed, or may be disposed at a position same as the position where the subjective optometry device is disposed. For example, when the operation portion is disposed at a position different from the position where the subjective optometry device is disposed, an operation portion connected to the second information processing device (for example, the operation portion 34B) may be used as the operation portion. **In** addition, for example, when the operation portion is disposed at a position same as the position where the subjective optometry device is disposed, an operation portion (for example, the operation portion 34A) connected to the first information processing device may be used as the operation portion. The configuration used as the operation portion is not limited to the above-described configuration, and different operation portions may be used.

For example, as the operation portion, at least one of user interfaces such as a touch panel, a mouse, a joystick, a keyboard, and a microphone may be used. For example, when the touch panel is used as the operation portion, the operation portion and a least a part of the display device may be shared. That is, the touch panel may be provided in the display area of the display device, and the touch panel can be used as the operation portion.

For example, when using the touch panel in the display area of the display device, the selection instruction to select the predetermined examination items, in the optometry flow displayed on the display device, may be input by operating the touch panel. For example, the selection instruction may be input such that the examination item, of the optometry flow displayed on the display device, corresponding to the position where the touch panel is operated is set as a predetermined examination item selected by the operator.

For example, when using a mouse or the like, an indicator (for example, a pointer, an icon, or the like) for selecting an examination item may be displayed in the display area of the display device. For example, the indicator may be information for emphasizing an operation position of the operation portion by the operator. In this case, for example, when the operation portion such as the mouse is operated by the operator, the indicator such as the pointer is moved in response to the operation of the operation portion. For example, the selection instruction may be input such that the examination item corresponding to the position of the indicator such as a pointer on the optometry flow is set as the predetermined examination item selected by the operator. For example, the indicator is not limited to the above-described configuration, and various indicators can be used. Lines and diagrams may be drawn and displayed as the indicator.

For example, the self-optometry assistance step may include a call receipt step and a call performing step. For example, in the call receipt step, an input of call performing instruction to perform the call action for calling the examiner who is the operator may be received. For example, in the call performing step, the call action may be performed based on the call performing instruction received in the call receipt step. For example, in a self-optometry assistance step, the input of the selection instruction, from the operation portion through which the examiner operates the subjective optometry device, may be restricted until the call performing instruction is input, and the restriction on the input from the operation portion may be released when the call performing instruction is input. With this configuration, for example, by restricting the selection operation from the operation portion used by the examiner, it is possible to prevent the examiner from touching the operation portion and interfering with the examination in the self-optometry.

For example, in the display control step, the examination item may be displayed on the display device as the optometry flow, the displayed examination item being limited to a predetermined examination item including at least the examination item being performed. With this configuration, for example, with regard to a plurality of examination items to be performed in the self-optometry, even if the number of optometry flows is large and it is difficult to check the plurality of examination items, it is easier to check the optometry flow for the current examination item and other examination items in a certain range, and thus, it becomes easier to grasp the current examination situation.

In this case, for example, in the display control step, when the number of a plurality of examination items displayed as the optometry flow exceeds the specified number of examination items, the examination items may be displayed on the display device as the optometry flow, the displayed examination items being limited to the predetermined examination items including at least the examination item being performed. In addition, in this case, for example, in the display control step, when the number of a plurality of examination items displayed as the optometry flow exceeds the number of examination items that can be displayed on the display device, the examination items is displayed on the display device as the optometry flow, the displayed examination items being limited to a predetermined examination item including at least the examination item being performed. With this configuration, for example, with regard to a plurality of examination items performed in the self-optometry, even if there are many examination items that exceed the number of examination items that can be displayed as the optometry flow, it is easier to check the optometry flow in a certain range for the current examination item and other examination items, and thus, it becomes easier to grasp the current examination situation in the optometry flow.

For example, when the examination item is displayed on the display device as the optometry flow, the displayed examination item being limited to the predetermined examination item, other examination items restricted to be displayed (examination items that are not displayed) may be displayed by the operation by the operator. For example, the examination items restricted to be displayed may be displayed by the operator operating the operation portion to perform a scroll operation (for example, a scroll operation in the vertical direction, a scroll operation in the horizontal direction, and the like). The method of displaying other examination items restricted to be displayed is not limited to the configuration described above, and various methods may be used. Other examination items restricted to be displayed may be displayed by the operation by the operator, and the selection instruction for the displayed other examination items is input, the examination of the examination items selected by the selection instruction may be performed.

For example, in the display control step, a predetermined examination item may be displayed on the display device as the optometry flow, the displayed predetermined examination item being limited to a predetermined examination item further including at least one of the examination item performed after the examination item being performed and the examination item performed before the examination item being performed. With this configuration, for example, since the examination item to be performed next (one item after) can be checked in addition to the examination item being performed, study for the optometry based on future examination (for example, the study with assuming the remaining time relating to the examination, the study whether the assistance is required in the next examination for the examinee who carries out the self-optometry, and the like) can be performed easily. In addition, for example, since the examination item performed before (one item before) can be checked in addition to the examination item being performed, study for the optometry based on the past examination situations (study for re-performing of the past optometry while taking the current examination situation into account, study of changing the procedure of self-examination based on the proceeding situation of past examination, and the like) can be performed easily. For example, the examination items before and after the examination item being performed can be checked in addition to the examination item being performed, it is easier to grasp the examination situation of the self-optometry, and thus, more detailed study regarding the examination can be performed easily.

For example, in the display control step, when a predetermined examination item is limited and displayed and when the call performing instruction is input, the limitation on the examination items displayed as the optometry flow may be released. With this configuration, for example, when the examiner is called, since the more examination items to be performed in the self-optometry are displayed in the optometry flow, the examiner can more easily grasp the examination situation in the self-optometry. As a result, it is easier to grasp the current situation of the self-optometry, and thus, it is possible to easily assist the self-optometry.

For example, when releasing the limitation on the display, the number of examination items displayed may be larger number than the number of restricted predetermined examination items. For example, the limitation on the display may be released such that one examination item is displayed in addition to the limited predetermined examination item. In addition, for example, the limitation on the display may be released such that all the examination items in a plurality of examination items are displayed. For example, since the entire examination items can be checked in the optometry flow, it is easier to grasp the examination situation.

For example, in the display control step, in the optometry flow, at least an identification, by which the examination item being performed can be identified, may be displayed. With this configuration, for example, the examination being performed can be easily grasped, and thus, it is possible to easily perform the study regarding the examination (for example, the necessity of redoing the examination and the necessity of changing the procedure of the self-optometry).

For example, the identification may be a display that emphasizes the examination item being performed. For example, a pointer, an icon, or the like may be displayed at the position of the examination item being performed. In addition, for example, the background at the position of the examination item being performed may be changed. In addition, for example, the size, color, or the like of the examination item being performed may be changed. In addition, for example, the examination item being performed may be emphasized by changing the display of other examination items except the examination item being performed. For example, the identification is not limited to the configuration described above, and various display methods can be used.

For example, with regard to the examination item on which identification is displayed, the identification may be displayed only on the examination item being performed. **In** addition, with regard to the identification which can be identified, the identification may be displayed on other examination items including the examination item being performed (for example, at least one of the examination item performed after the examination item being performed, the examination item performed before the examination item being performed, and the like).

For example, for the self-optometry assistance step, at least one of a plurality of examination items and the procedure of the self-optometry can be changed according to the instructions input by the operator. **In** the display control step, the optometry flow may be changed and displayed on the display device based on at least one of the plurality of examination items and the procedure changed according to the instructions input by the operator. For example, the change of a plurality of examination items may be at least one of an addition of any examination item and deleting any examination item. For example, with this configuration, since the examination items and the procedure can be changed appropriately, and the optometry flow corresponding to the change of the procedure can be displayed, it is possible to carry out the self-optometry considering intention of the examiner. **In** addition, for example, the self-optometry in accordance with the examinee becomes possible, and it is possible to proceed with the optometry smoothly.

For example, with regard to a plurality of examination items in the optometry flow, the information relating to each examination item may be changed. For example, a name of the examination item may be changed. In addition, for example, the target and the calibration optical system used when performing the examination of the examination item may be changed. For example, the change of the information relating to the examination item is not limited to the configuration described above, any settings relating to the examination item (performed examination of the examination item) may be changed.

For example, the self-optometry assistance step may be performed according to the instruction input to the second information processing device which is another information processing device connected to the first information processing device via a network. With this configuration, even if the examiner is in a remote location that is different from the place of examinee, the examiner can appropriately assist the self-optometry by examinee from the remote location by inputting the instruction to the second information processing device.

When there is a problem in the proceeding of the self-optometry (for example, when the instruction to perform the assistance action is input, and the like), the control portion of the first information processing device may output an instruction, to the second information processing device, to perform the calling action for calling the examiner. In this case, even when the examiner is in a remote location different from the place of examinee, the examiner can easily grasp that there is a problem in the proceeding of the self-optometry.

However, the self-optometry assistance step may be performed according to an instruction directly input to the first information processing device (for example, an instruction input to the operation portion of the first information processing device). That is, the examiner at the place where the subjective optometry device and the first information processing device are disposed may input the instruction to perform the self-optometry assistance step.

In addition, when the second information processing device is used, the number of second information processing devices may be one, or two or more. In addition, similarly to the first information processing device, various devices (for example, a PC, a mobile terminal, a smartphone, and the like) can be used as the second information processing device. The second information processing device may have been connected to the first information processing device already when the self-optometry is carried out, or may be connected to the first information processing device after a problem occurs in the self-optometry.

In addition, when the second information processing device is used, each control portion of the first information processing device and the second information processing device may perform a remote conversation step of assisting a conversation between the examiner and the examinee by transmitting and receiving at least audio data (which may be both the audio data and image data). In this case, the examiner at the remote location can appropriately assist the self-optometry by grasping the situation of the examinee through the conversation.

### <Example>

Hereinafter, one of typical examples in the present disclosure will be described with reference to the drawings. As shown in FIG. 1, a subjective optometry system 100 in the present example includes a subjective optometry device 1 and a first information processing device 2A. The subjective optometry device 1 is used for subjectively measuring optical characteristics of a subject eye. The optical characteristics of the subject eye measured by the subjective optometry device 1 in the present example is an eye refractive power. The measured eye refractive power may be at least one of a spherical power, a cylindrical power, an astigmatic axis angle, and the like of the subject eye. The first information processing device 2A is connected to the subjective optometry device 1. Hereinafter, the first information processing device 2A may be referred to as a connection device. In addition, the first information processing device 2A is connected to a second information processing device 2B which is another information processing device, via a network 5. That is, the first information processing device 2A in the present example may be remotely accessed by the second information processing device 2B. Hereinafter, each device will be described in detail.

The subjective optometry device 1 will be described. The subjective optometry device 1 includes an eye refractive power measurement unit 10, a target presentation unit 15, and a relay portion 19.

The eye refractive power measurement unit 10 includes a calibration optical system 11 and a drive portion 12. The calibration optical system 11 changes the optical characteristics of the target light flux presented to the subject eye. That is, the calibration optical system 11 changes at least one of the spherical power, the cylindrical power, the astigmatic axis angle, polarization characteristics, and an amount of aberration of the target light flux. For example, the calibration optical system 11 in the present example changes the optical characteristics of the target light flux by switching the optical element, among a plurality of optical elements, disposed at an examination window in front of the subject eye. In the calibration optical system 11 in the present example, a left eye lens disc and a right eye lens disc in which a plurality of optical elements are arranged on the same circumference are used. Each of the left eye lens disc and the right eye lens disc may be one or more. As the optical element, for example, at least one of a spherical lens, a cylindrical lens, a cross cylinder lens, a rotary prism, a wave surface modulation element, and the like may be used. The drive portion 12 changes the optical characteristics of the target light flux by driving the calibration optical system 11. The drive portion 12 in the present example drives the calibration optical system 11 by rotating each of the left eye lens disc and the right eye lens disc and switching the optical element to be disposed at the examination window. For example, a step motor or the like can be used as the drive portion 12. The drive portion 12 is driven in response to the drive signal.

The target presentation portion 15 presents an examination target (for example, at least one of a Landolt ring, characters, and the like) on the subject eye, and switches the examination target to be presented to the subject eye. Specifically, the target presentation portion 15 includes a target presentation portion 16 and a drive portion 17. The target presentation portion 16 presents the examination target to the subject eye. For example, at least one of a space-saving type target projection device that projects the examination target onto the subject eye via a concave mirror, a chart projector that projects the examination target onto the screen, a display that displays the examination target, and the like can be used as the target presentation portion 16. The target presentation portion 16 is disposed at substantially the same height as the eye refractive power measurement unit 10 such that a distance from the subject eye is optically a predetermined distance. The drive portion 17 switches the examination target to be presented to the subject eye by driving the target presentation portion 16. The drive portion 17 is driven in response to the drive signal.

The relay portion 19 relays the drive signal between the first information processing device 2A and the drive portions 12 and 17. In the present example, a system of the drive signal output from the first information processing device 2A and a system of the drive signal that can control at least one of the drive portions 12 and 17 are different from each other. The relay portion 19 in the present example converts the drive signal received from the first information processing device 2A into a drive signal that can control the drive portions 12 and 17, and transmits the drive signal to the drive portions 12 and 17. Furthermore, for example, when receiving one drive signal for driving two drive portions 12 and 17 from the first information processing device 2A, the relay portion 19 in the present example converts the received one drive signal into two drive signals for driving each of the two drive portions 12 and 17, and transmits the two drive signals to each of the two drive portions 12 and 17.

The first information processing device 2A and the second information processing device 2B will be described. At least any one of various information processing devices that can process various information can be used as the first information processing device 2A and the second information processing device 2B. For example, a personal computer (hereinafter, referred to as a "PC") can be used as the first information processing device 2A and the second information processing device 2B in the present example. However, the information processing device that can function as the first information processing device 2A and the second information processing device 2B in the present example is not limited to the PC. For example, a server, a mobile terminal, a smartphone, or the like may be used as at least one of the first information processing device 2A and the second information processing device 2B. At least one of the first information processing device 2A and the second information processing device 2B may be configured with a plurality of devices. For example, the first information processing device 2A may be configured with a dedicated controller including a controller and a storage device, and a personal computer.

The first information processing device 2A and the second information processing device 2B are connected to each other in a communicable state, via a network 5 (for example, the Internet). In the example shown in FIG. 1, a case where a plurality of the second information processing devices 2B are connected to one first information processing device 2A is illustrated. However, one second information processing device 2B may be connected to one first information processing device 2A. In addition, one second information processing device 2B may be connected to a plurality of the first information processing devices 2A.

The first information processing device 2A is disposed at a position (for example, an optician, a hospital, or the like) where the subjective optometry for the examinee is carried out. The first information processing device 2A includes a CPU 21A and a storage device 22A. The CPU 21A is a control portion (controller) that controls the first information processing device 2A. The storage device 22A can store programs, various data, and the like. In the present embodiment, an optometry control program is stored in the storage device 22A.

The first information processing device 2A is connected to the subjective optometry device 1 (specifically, the relay portion 19 of the subjective optometry device 1) in a communicable state. As a connection standard between the first information processing device 2A and the subjective optometry device 1, various standards such as LAN can be used. In addition, the first information processing device 2A is connected to an objective optometry device 3 in a communicable state. The objective optometry device 3 objectively measures the optical characteristics of the subject eye (for example, at least one of the spherical power, the cylindrical power, and the astigmatic axis angle). As the connection standard between the first information processing device 2A and the objective optometry device 3, various standards such as LAN can be used. The objective optometry device 3 may be connected to the relay portion 19. A result of measurement by the objective optometry device 3 may be stored in the storage device included in the relay portion 19.

A camera 31A, a microphone 32A, a speaker 33A, an operation portion 34A, and a display portion 35A are connected to the first information processing device 2A. The camera 31A captures an image. In particular, the camera 31A in the present embodiment is used for capturing a moving image of the examinee. The microphone 32A converts sound into an audio signal to output. The speaker 33A converts the audio signal into sound. The operation portion 34A is operated by the user (for example, examinee, and the like) for inputting various instructions. As the operation portion 34A, for example, at least one of a keyboard, a mouse, a touch panel, and the like may be used. As the operation portion 34A, a dedicated operation portion (for example, a joystick or the like) suitable for inputting a response of the subjective optometry may be used. The display portion 35A displays various images. Various devices capable of displaying images (for example, at least one of a monitor, a display, a projector, and the like) can be used as the display portion 35A.

The second information processing device 2B is disposed at the position of the examiner (for example, an optician clerk, a doctor, or a nurse who is familiar with optometry using the subjective optometry device 1) capable of proceeding with the optometry using the subjective optometry device 1. The second information processing device 2B includes a CPU 21B and a storage device 22B. The CPU 21B is a control portion (controller) that controls the second information processing device 2B. The storage device 22B can store programs, various data, and the like.

A camera 31B, a microphone 32B, a speaker 33B, an operation portion 34B, and a display portion 35B are connected to the second information processing device 2B. As these devices described above, similar to the devices connected to the first information processing device 2A, various devices described above can be used.

### <Program and Optometry Method>

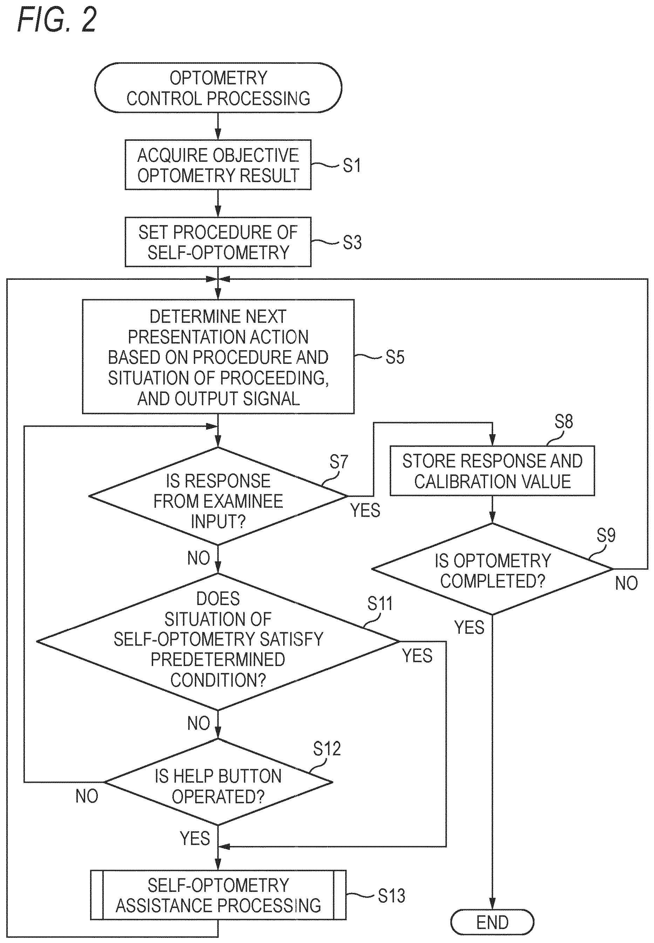

The program installed in the first information processing device 2A in the present example will be described. As described above, an optometry control program for performing the optometry control processing (see FIG. 2) is stored in the storage device 22A included in the first information processing device 2A. The optometry control program includes a drive control program for performing a drive control and a self-optometry program for carrying out a self-optometry. The drive control program transmits a control signal for controlling the drive of the subjective optometry device 1 to the subjective optometry device 1. The self-optometry program automatically proceeds with the optometry by the subjective optometry device 1 according to the response input by the examinee. The drive control program for performing the drive control and the self-optometry program for carrying out the self-optometry may be built separately, or may be incorporated into one program.

A subjective optometry method that can be performed by the subjective optometry system 100 in the present example will be described. The subjective optometry system 100 in the present example can carry out the self-optometry and the remote optometry. The self-optometry is the optometry carried out by the self-optometry program. That is, in the self-optometry, the optometry is automatically proceeded based on the response input by examinee. In addition, in the subjective optometry system 100 in the present example, when a problem occurs in the proceeding of the self-optometry, the self-optometry assistance processing for assisting the proceeding of the self-optometry is performed. The self-optometry assistance processing can also be performed according to the instruction signal input to the second information processing device 2B at the remote location. Therefore, even if a problem occurs in the proceeding of the self-optometry, the optometry can be carried out smoothly. The remote optometry is an optometry carried out according to an instruction signal input to the second information processing device 2B. Hereinafter, the explanation will be focused on the self-optometry.

### <Optometry Control Processing>

An example of optometry control processing performed by the first information processing device 2A of the subjective optometry system 100 in the present example will be described with reference to FIG. 2 to FIG. 4B. In the optometry control processing, for example, processing for controlling the self-optometry, processing for assisting the self-optometry, and the like are performed. When the instruction to start the subjective optometry for the subject eye is input to the first information processing device 2A, the CPU 21A of the first information processing device 2A performs the optometry control processing shown in FIG. 2 according to the optometry control program.

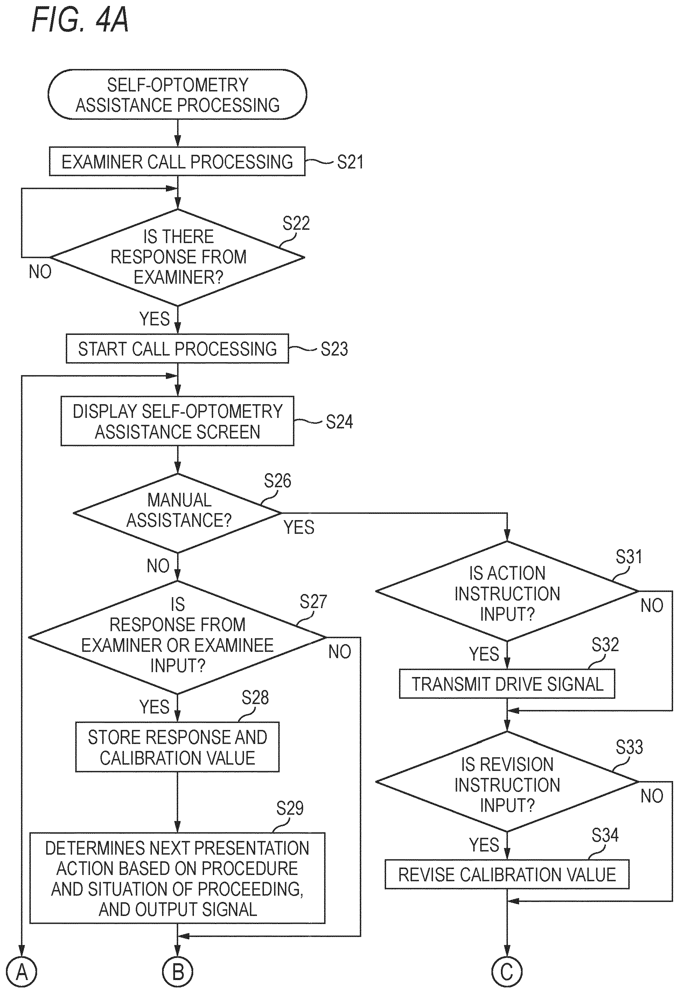

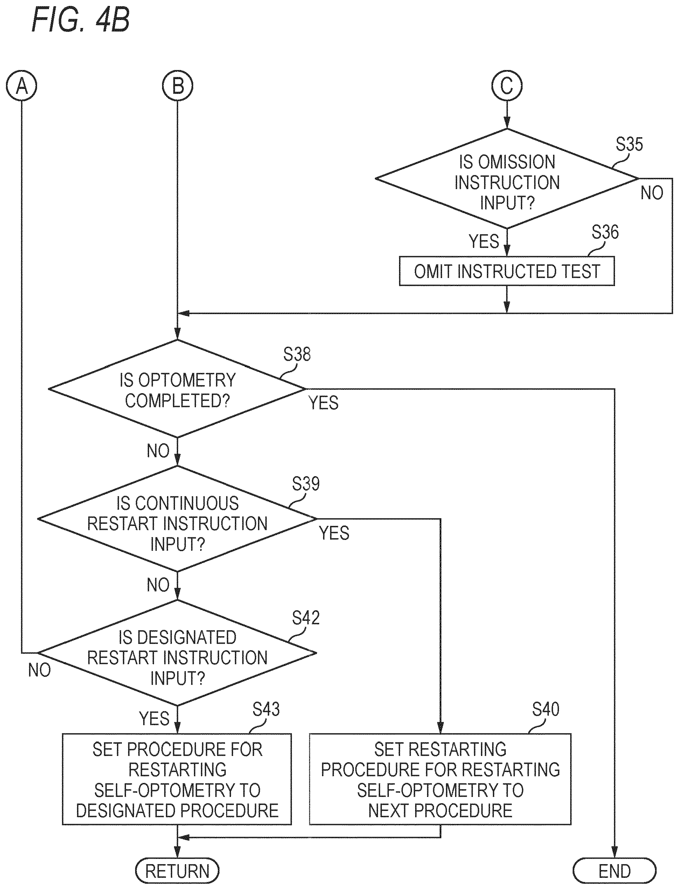

For example, the optometry control processing in the present example is performed in a state where the communication (for example, remote access or the like) of one or a plurality of the second information processing device 2B to the first information processing device 2A is established. However, the communication between the first information processing device 2A and the second information processing device 2B may be established during the performance of the optometry control processing (for example, at the time of starting the self-optometry assistance processing shown in FIGs. 4A and 4B). The communication method between the first information processing device 2A and the second information processing device 2B can be appropriately selected. For example, by using a remote access service (RAS), a remote access of the second information processing device 2B to the first information processing device 2A may be established.

First, the CPU 21A acquires an objective optometry result (S1). As an example, the first information processing device 2A in the present example acquires the objective optometry result of the same examinee from the objective optometry device 3 (refer to FIG. 1) connected via LAN or the relay portion 19. However, the CPU 21A may acquire the objective optometry result of the same examinee, for example, from a detachable memory, the network 5, or the like. The objective optometry result may be input by the user from the operation portion 34A or the like. If there is no objective optometry result of the same examinee, the processing of S1 may be omitted.

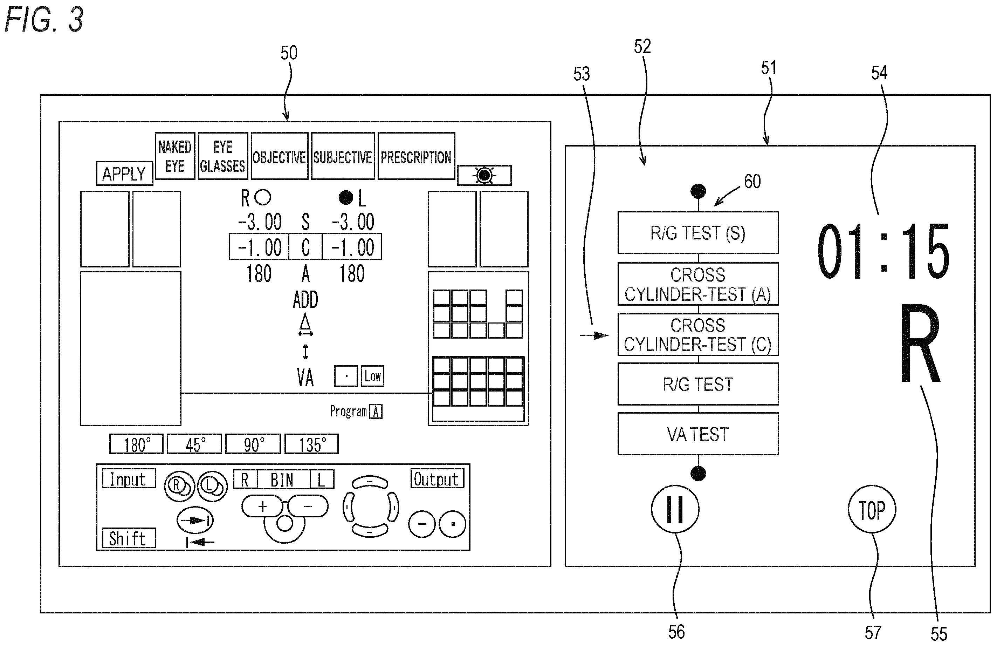

Next, the CPU 21A sets the procedure of the self-optometry (S3). In a procedure display field 52 in FIG. 3, a part of an example of the procedure of the self-optometry used in the subjective optometry system 100 in the present example (in FIG. 3, only the procedure for the right eye) is displayed. As shown in FIG. 3, the procedure used in the present example defines a plurality of tests (a plurality of examinations) performed for the examinee and a performing order of the plurality of tests. For example, in the procedure shown in FIG. 3, after the R/G test (S), cross cylinder-test (A), cross cylinder-test (C), R/G test, and VA test for the right eye of the examinee are performed, R/G test (S), cross cylinder-test (A), cross cylinder-test (C), R/G test, and VA test for the left eye of the examinee are performed. In the R/G test (S), the spherical power of the subject eye is measured. In the cross cylinder-test (A), the astigmatic axis angle of the subject eye is measured. In the cross cylinder-test (C), the astigmatic power of the subject eye is measured. In the R/G test, it is confirmed whether the adjustment by the subject eye is working or not in the performed test. In the VA test, the maximum visual acuity of the subject eye is measured.

When the objective optometry result of the same examinee is acquired in S1, the procedure of the self-optometry is set according to the objective optometry result (for example, eye refractive power measured for the same subject eye (at least one of the spherical power, astigmatic power, and astigmatic axis angle)) in S3. For example, in each test included in the procedure, the optical element to be disposed first in the examination window of the calibration optical system 11, and the type and size of the target to be presented to the target presentation portion 16 may be set according to the objective optometry result. In addition, if the astigmatic power obtained by the objective optometry is equal to or less than a threshold value, the procedure in which the test relating to the astigmatism (for example, the cross cylinder-test (A) and the cross cylinder-test (C)) is omitted may be set. When the objective optometry result is not acquired in S1, a default procedure may be set in S3.

Next, the CPU 21A determines a presentation action of the target, which the subjective optometry device 1 performs next, according to the procedure set in S3 and the situation of proceeding of the self-optometry, and outputs a presentation instruction signal (drive signal) for performing the determined presentation action to the subjective optometry device 1 (S5). Specifically, in S5 in the present embodiment, at least one of the optical element to be disposed in the examination window of the calibration optical system 11 and the target to be presented by the target presentation portion 16 is determined as the next presentation action. The drive signal for at least one of the drive portion 12 and the drive portion 17 for performing the determined action is transmitted to the subjective optometry device 1. When transmitting the drive signal to the subjective optometry device 1, the CPU 21A outputs a guidance voice, from the speaker 33A, according to the content of examination. Therefore, the examinee can see the presented examination target while appropriately understanding the content of examination.

In the present example, the drive signal is transmitted from the first information processing device 2A to the drive portions 12 and 17 via the relay portion 19 (refer to FIG. 1) described above. Therefore, the signal transmitted from the first information processing device 2A does not need to go through a dedicated controller or the like. Accordingly, since a signal processing by the dedicated controller and the like is omitted, the optometry is carried out more smoothly.

In the self-optometry, the examinee visually recognizes the examination target presented by the subjective optometry device 1 and inputs a response of the visually recognized result to the first information processing device 2A while understanding the content of examination by the guidance voice. The response is input by operating the dedicated operation portion 34A configured to input the response of the subjective optometry by the examinee. The response is input by the operation portion 34A (for example, a mouse, a touch panel, a keyboard, and the like). In addition, the response may be input by the audio signal converted by the microphone 32A.

The CPU 21A determines whether or not the response of the examinee is input (S7). When the response is input (YES in S7), the input response and the optical specific calibration value (measured value) of the subject eye are stored in the storage device 22A (S8). The calibration value of the optical characteristics of the subject eye is acquired based on the response input in S7, the target presented to the subject eye when the response is input, and the optical characteristics of the optical element disposed at the examination window of the calibration optical system 11 (that is, the optical characteristics of the target light flux).

Next, if the series of self-optometry is not completed yet (NO in S9), the process returns to S5 and the self-optometry is continued along to the procedure. For example, in the VA test in the present example, when the response of the examinee acquired in S7 is a correct response, the CPU21A determines the next target presentation action such that the target to be presented by the target presentation portion 16 becomes a target having a visual acuity value one step higher than that of the target presented last time (for example, a target of which the size is one step smaller). When the response of the examinee acquired in S7 is an incorrect response, the CPU 21A determines the next target presentation action such that the target to be presented in the target presentation portion 16 becomes a target having a visual acuity value one step lower than that of the target presented last time (for example, a target of which the size is one step larger). The CPU 21A may determine the calibration power of the optical element to be disposed at the examination window of the calibration optical system 11 as the next target presentation action together with switching of the target.

When the processes of S7 to S9 are repeatedly performed and a series of the self-optometry is completed successfully (YES in S9), the optometry control processing ends.

When the response of the examinee is not input (NO in S7), it is determined whether or not a predetermined condition in which the situation of the self-optometry is likely to be inappropriate is satisfied (S11). For example, in the present embodiment, when a predetermined time elapses without a response of the examinee being input after the presentation instruction signal is transmitted in S5, it is determined that the predetermined conditions are satisfied. In addition, when the response input by the examinee is inappropriate (for example, when the response different from the response candidate requested by the examinee is input, when the same response is input more than a predetermined number of times consecutively, when the responses are input more than the number of responses requested to the examinee, or the like), it is also determined that the predetermined condition is satisfied. When it is determined that the predetermined condition is satisfied (YES in S11), the self-optometry assistance processing is performed (S13).

When the response of the examinee is not input (NO in S7) and when the self-optometry condition does not satisfy the condition (NO in S11), the CPU 21A determines whether or not the instruction to perform the assistance action for the self-optometry is input by the examinee (S12). For example, in the present embodiment, the examinee can input the instruction to perform the assistance action for the self-optometry by operating a help button 70 provided on the dedicated operation portion 34A or a help button displayed on the display portion 35A, and the like. If the instruction to perform the assistance action is not input (NO in S12), the process returns to S7. When the instruction to perform the assistance action is input (YES in S12), the self-optometry assistance processing is performed (S13).

The details of the self-optometry assistance processing will be described with reference to FIGs. 4A and 4B. First, the CPU 21A performs the examiner call processing (S21). As an example, in S21 in the present embodiment, the CPU 21A transmits an examiner call instruction, via the network 5, to one or a plurality of the second information processing devices 2B of which the remote access to the first information processing device 2A is established (S4). The examiner call instruction is an instruction for causing the second information processing device 2B to perform the calling action for calling the examiner to request the examiner to assist the self-optometry. The calling action may be performed by at least one method such as the audio output and the image display. By performing the calling action, the user (examiner) of the second information processing device 2B can appropriately grasp the fact that the assistance for the self-optometry (remote assistance in the present embodiment) is required.

Next, the CPU 21A determines whether or not there is a response of the user (for example, the examiner or the like) of the second information processing device 2B (S22). When there is no response of the examiner of any of the second information processing devices 2B (NO in S22), since it is impossible to perform the assistance for the self-optometry by the examiner, the determination in S22 is repeated to enter the standby state. When the user of any of the second information processing devices 2B is in a state of being able to assist the self-optometry, and the response instruction is input to the second information processing device 2B (YES in S22), the process proceeds to S23.

Next, the CPU 21A starts call processing by starting transmission and reception of voice data to and from the second information processing device 2B used by the examiner who assists the self-optometry (S23). As a result, the assistance for the self-optometry processing described below is performed while the examinee and the examiner can have a conversation. Specifically, the CPU 21A transmits audio data input from the microphone 32A to the second information processing device 2B. In addition, the CPU 21A receives audio data input, from the microphone 32B, to the second information processing device 2B and outputs the audio data to the speaker 33A. The CPU 21A may transmit and receive image data to and from the second information processing device 2B together with the audio data. In this case, the CPU 21A may transmit the image data input from the camera 31A to the second information processing device 2B. In addition, the CPU 21A may receive the image data input, from the camera 31B, to the second information processing device 2B and display the image data on the display portion 35A.

Next, the CPU 21A displays the self-optometry assistance screen (refer to FIG. 3) on the display portion 35B of the second information processing device 2B used by the examiner assisting the self-optometry (S24). As shown in FIG. 3, the self-optometry assistance screen in the present example includes an operation image area 50 and a procedure display area 51.

In the operation image area 50, an operation image including information on the optical characteristics of the target light flux presented to the subject eye is displayed. In the operation image of the present example, the value relating to the optical characteristics of the target light flux presented to the subject eye is displayed for each type of optical characteristics. The examiner can instruct a value for a desired type among a plurality of optical characteristics (the spherical power, the cylindrical power, the astigmatic axis angle, and the like) by operating various buttons and values on the operation image area 50 via the operation portion such as a touch panel or a mouse. When the optometry is appropriately carried out, the calibration value of the optical characteristics displayed in the operation image becomes the measured value of the optical characteristics of the subject eye.

In the present example, the procedure display area 51 is displayed on the display portion 35A connected to the first information processing device 2A, in addition to the display portion 35B connected to the second information processing device 2B. In the procedure display area 51 of the present example, a procedure display field 52, an elapsed time display field 54, a left and right eye display field 55, a help button 56, and a top button 57 are provided. As described above, in the procedure display field 52, the optometry flowchart 60 (hereinafter, referred to as optometry flow) of the procedure set for the self-optometry being carried out is displayed (details will be described later). In the example shown in FIG. 3, the identification, in which the procedure being performed at that time (examination item being performed) among the entire procedure (that is, a plurality of tests included in the procedure) can be identified, is displayed. In the present example, as the identification, the examination item being performed is displayed on the procedure by an arrow 53 and a thick frame.

In the elapsed time display field 54, an elapsed time from beginning of the self-optometry being carried out is displayed. In the left and right eye display field 55, whether the procedure being performed at that time, among the entire procedure, is the procedure to be performed for the left eye or the right eye of the examinee is displayed. The help button 56 is operated by the examinee when the examinee inputs the instruction to perform the assistance action for the self-optometry. The top button 57 is operated when the screen displayed in the procedure display area 51 is returned to a top screen of the self-optometry program.

During the assistance for the self-optometry (S24 to S43), which will be described later, the audio signal input from the microphone 32A to the first information processing device 2A is converted into a voice by the speaker 33B of the second information processing device 2B as described above. In addition, the audio signal input from the microphone 32B to the second information processing device 2B is converted into the voice by the speaker 33A of the first information processing device 2A. Therefore, the examiner and the examinee can have a conversation during the assistance for the self-optometry. During the assistance for the self-optometry, the examiner using the second information processing device 2B can input various instructions for assisting the self-optometry from at least one of the operation portion 34B and the microphone 32B.

Next, the CPU 21A determines whether or not the manual assistance by the examiner is being performed (S26). In the present example, any one of an indirect assistance or the manual assistance is selected as the method for the examiner to assist the self-optometry. The indirect assistance is a method in which the examiner indirectly assists the self-optometry while proceeding the self-optometry carried out by the self-optometry program along the procedure set in S3. The manual assistance is a method of assisting the self-optometry by proceeding the optometry according to the instructions input by the examiner, instead of the proceeding of the self-optometry carried out along the procedure.

The examiner can cause the first information processing device 2A, using various methods, to perform any one of the indirect assistance or the manual assistance. For example, in the present example, at the beginning of the self-optometry assistance processing, the indirect assistance is set in advance so as to be performed first. In addition, by inputting an action instruction via various buttons on the operation image area 50, a revision instruction for the calibration value, or an omission instruction for the procedure is input to the subjective optometry device 1, the indirect assistance can be switched to the manual assistance. However, the method of switching between the indirect assistance and the manual assistance can be appropriately selected. For example, a button for switching between the indirect assistance and the manual assistance may be provided on the self-optometry assistance screen.

If the indirect assistance is being performed (NO in S26), the CPU 21A determines whether or not the response of the examinee who visually recognizes the presented target is input by the examiner or the examinee (S27). In the present example, during the performance of the indirect assistance, the examiner can input the response heard from the examinee to the second information processing device 2B by operating the operation portion 34B connected to the second information processing device 2B. The response input to the second information processing device 2B is transmitted to the first information processing device 2A. Therefore, even if the examinee does not grasp the method and the like of response in the self-optometry, the proceeding of the self-optometry is appropriately assisted. In addition, in the present example, even during the performance of the indirect assistance, the examinee can input the response for the result of visually recognizing the target by himself, similarly to the case of S7 (refer to FIG. 2) described above. Accordingly, when receiving an advice from the examiner and grasping the method of response, and the like, the examinee can also input the response by himself to proceed with the optometry procedure. However, during the performance of the indirect assistance, one of the first information processing device 2A and the second information processing device 2B may receive the response.

When the response of the examinee is not input to any one of the first information processing device 2A and the second information processing device 2B (NO in S27), the process proceeds to S38. When the response of the examinee is input to the first information processing device 2A or the second information processing device 2B (YES S27), the input response and the optical characteristics measured value of the subject eye are stored in the storage device 22A, similarly to the case of S8 (refer to FIG. 2) (S28). Next, the CPU 21A determines the presentation action of the target which the subjective optometry device 1 performs next according to the procedure set in S3 (refer to FIG. 2) and the proceeding situation at that time, and outputs the presentation instruction signal (drive signal), to the subjective optometry device 1, for performing the determined presentation action (S29). That is, the self-optometry carried out by the self-optometry program along the procedure is continued. After that, the process proceeds to S38.

In addition, if the manual assistance is being performed instead of the indirect assistance (S26: YES), the CPU 21A determines whether or not the action instruction for the examiner to manually proceed at least a part of the procedure is input by the examiner (S31). The examiner can determine the action instruction (that is, at least one of the instruction for the dispose action of the optical element in the calibration optical system 11 and the instruction for the presentation action of the target in the target presentation portion 16) to the subjective optometry device 1 based on, for example, the displayed content on the self-optometry assistance screen (refer to FIG. 3) or the conversation with the examinee. In this case, the examiner inputs the determined action instruction to the second information processing device 2B via the operation portion 34B or the like. The input action instruction is acquired by the first information processing device 2A via the network 5. When the action instruction is input and acquired (YES in S31), the CPU 21A transmits the presentation instruction signal (drive signal) to the subjective optometry device 1 for causing the subjective optometry device 1 to perform the instructed action (S32).

Next, the CPU 21A determines whether or not the revision instruction for the calibration value stored in S8 (refer to FIG. 2) or S28 is input by the self-optometry (S33). When it is determined that the revision of the calibration value (measurement value) displayed on the self-optometry assistance screen (refer to Fig. 3) is necessary, the examiner inputs the revision instruction for the calibration value that requires the revision, to the second information processing device 2B via the operation portion 34B or the like. The input revision instruction is acquired by the first information processing device 2A via the network 5. When the revision instruction is input and acquired (YES in S33), the CPU 21A revises the calibration value according to the instruction (S34).

Next, the CPU 21A determines whether or not the omission instruction for at least a part of the procedure (at least one of a plurality of tests in the present embodiment) is input (S35). When the omission instruction is input via the second information processing device 2B and the network 5 (YES in S35), the CPU 21A omits the instructed test from the procedure among the plurality of tests included in the procedure (S36). After that, the process proceeds to S38.

Next, the CPU 21A determines whether or not a series of optometry for the examinee is completed (S38). For example, the CPU 21A may determine that a series of optometry is completed if the self-optometry carried out along the procedure is completed. In addition, the CPU 21A may determine that a series of optometry is completed if an instruction to end the optometry is input to the examinee. When a series of optometry is completed (YES in S38), the process ends.

Next, the CPU 21A determines whether or not a continuous restart instruction for the self-optometry is input (S39). The continuous restart instruction is an instruction to restart the self-optometry from the procedure next to the procedure which has already been completed among the procedure set in S3 (refer to FIG. 2). For example, the CPU 21A may determine that the continuous restart instruction is input when a continuous restart button (not illustrated) is operated by the examinee. When the continuous restart instruction is input via the second information processing device 2B and the network 5 (YES in S39), the CPU 21A sets the restarting procedure for restarting the self-optometry to the procedure next to the procedure which has already been completed among the procedure (S40), and the process returns to the optometry control processing (refer to FIG. 2). In the processing in S5 to S9, the self-optometry is restarted from the restarting procedure set in S40. If the continuous restart instruction is not input (NO S39), the process proceeds to S42.

Next, the CPU 21A determines whether or not designated restart instruction for the self-optometry is input (S42). The designated restart instruction is an instruction to restart the self-optometry from the procedure designated by the examiner among the procedure set in S3 (refer to FIG. 2). In the present embodiment, the examiner can input the designed restart instruction by designating the restarting procedure for restarting the self-optometry on the procedure displayed in the procedure display field 52 (refer to FIG. 3) of the self-optometry assistance screen. For example, in the present example, the examiner inputs a designated restart instruction using a mouse or a touch panel. For example, the optometry flow may be used to designate the restarting procedure for restarting the self-optometry (details will be described later). A designated restart instruction button (reset button) for erasing the results of self-optometry that has already been carried out along the procedure and restarting the self-optometry from the beginning of the procedure may be provided in the self-optometry assistance screen or the like. When the designated restart instruction is input via the second information processing device 2B and the network 5 (YES in S42), the CPU 21A sets the restarting procedure for restarting the self-optometry to the procedure designated by the designed restart instruction among the procedure (S43), and the process returns to the optometry control processing (refer to FIG. 2). In the processing in S5 to S9, the self-optometry is restarted from the restarting procedure set in S43.

The technology disclosed in the present example described above is just an example. Therefore, the technology exemplified in the present example can be modified. For example, only a part of the technology illustrated in the present example described above may be performed. For example, if there is a problem in the self-optometry (YES in S11 or YES in S12), the subjective optometry system 100 in the present example described above performs both the examiner call processing (S21) and the assistance for the self-optometry processing by the examiner (S23 to S43). However, the subjective optometry system 100 may perform only one of the examiner call processing or the assistance processing by the examiner. For example, even if the examiner is called by the examiner call processing, the self-optometry will be appropriately assisted.

In the present example described above, the self-optometry is started in a state where the second information processing device 2B is connected to the first information processing device 2A. However, after a problem occurs in the proceeding of the self-optometry (YES in S11 or YES in S12), the second information processing device 2B may be connected to the first information processing device 2A.

The self-optometry assistance processing (refer to FIGs. 4A and 4B) in the present example is performed according to the instruction input to the second information processing device 2B disposed at the remote location. However, the self-optometry assistance processing may be performed according to an instruction directly input to the first information processing device 2A (for example, an instruction input to the first information processing device 2A by the examiner operating the operation portion 34A). That is, the examiner at the place where the subjective optometry device 1 is disposed may input the instruction for assisting the self-optometry. In addition, both the first information processing device 2A and the second information processing device 2B may receive the input of the instruction for assisting the self-optometry. In this case, the examiner can assist the self-optometry at any of the places where the subjective optometry device 1 is disposed or the remote location. When the instruction for assisting the self-optometry is input via the first information processing device 2A, the processing for calling the examiner in S21 (refer to FIG. 4A) may be performed in the first information processing device 2A or may be performed in both the first information processing device 2A and the second information processing device 2B.

In S5 in FIG. 2 and S29 in FIG. 4A, the processing for outputting the presentation instruction signal to the subjective optometry device 1 based on the procedure is an example of a "self-optometry proceeding step". The processing for acquiring the response from the examinee in S7 in FIG. 2 and S27 in FIG. 4A is an example of a "response acquisition step". The processing for storing the calibration value in S8 in FIG. 2 and S28 in FIG. 4A is an example of a "calibration value storage step". The self-optometry assistance processing shown in FIGs. 4A and 4B is an example of a "self-optometry assistance step". The processing for outputting the presentation instruction signal according to the action instruction in S31 and S32 in FIG. 4A is an example of a "manual processing step". The processing for revising the calibration value in S33 and S34 in FIG. 4A is an example of a "calibration value revision step". The processing for omitting the procedure in S35 and S36 in FIG. 4B is an example of a "procedure omission step". In S27 in FIG. 4A, the processing for acquiring the response of examinee according to the instruction input by the examiner is an example of a "proxy response acquisition step". The processing for restarting the self-optometry from the next procedure in S39 and S40 in FIG. 4B is an example of a "continuous restart step". In S42 and S43 in FIG. 4B, the processing for restarting the self-optometry from the procedure designed by the examiner is an example of a "designated restart step". The processing for restarting the self-optometry in S33 and S34 in FIG. 4A is an example of a "self-optometry restart step". The processing for displaying the procedure in S24 in FIG. 4A is an example of a "procedure display step".

### < Self-Optometry Assistance based on Optometry Flow>

Hereinafter, an example of performing the assistance action using the self-optometry assistance screen (refer to FIG. 3) will be described. First, an action for performing the self-optometry assistance will be described. For example, when the help button 56 is operated by the examinee, the CPU 21A receives the input of call performing instruction and performs the examiner call action.

As the examiner call action, the CPU 21A may display, for example, a call display for calling the examiner on a display device (for example, at least one of the display portion 35A and the display portion 35B). A display form of the call display may be, for example, a message for calling the examiner (for example, HELP display), a mark for calling the examiner, or the like. In addition, a display form of the call display is not limited to this, and various modifications such as blinking the display screen and changing the background of the display screen may be used.

In this case, a display device for displaying the call display is not limited to the display portion 35A and the display portion 35B, but may be a stationary display (a display other than the display portion 35A) disposed in a place having the subjective optometry device, or may be a tablet or smartphone display (a display other than the display portion 35A) owned by a staff in the place having the subjective optometry device. Even a display other than the display portion 35B used in the remote location can be applied to the present example. Needless to say, the display device is not limited to a general-purpose display, and a display provided on a dedicated controller for the examiner operated by the examiner may be used.

In addition, as the examiner call action, the CPU 21A may generate, for example, a calling voice for calling the examiner via a voice generation portion. A form of the voice may be, for example, a voice to call the name of a specific examiner, or may be a voice for the examiner to move to the subjective optometry device. In addition, the form of the voice is not limited to this, and a simple beep sound may be output according to the call performing instruction.

For example, with regard to a procedure for performing assistance action by the examiner, for example, when the examinee performs a notification action for calling the examiner and the examiner is ready to perform the optometry assistance for the examinee, the examiner operates a response switch that corresponds to the optometry assistance. When the response switch is pressed, the CPU 21A ends the examiner call action. For example, as the response switch, the help button 56 may also be used as shown in FIG. 3, and when the help button 56 is touched, a response signal by the examiner may be input. Of course, the form of the response switch is not limited to this, and a physical switch may be provided.

For example, by operating the response switch, the examiner is in a state of being able to operate the subjective optometry device 1. When the input of call performing instruction is received, the examiner may be in a state of being able to operate the subjective optometry device 1.

In the description below, a case where the operation portion 34B connected to the second information processing device 2B is used as the operation portion for performing the self-optometry assistance will be described as an example. For example, as the operation portion 34A connected to the first information processing device 2A may be used as the operation portion for performing the self-optometry assistance. The configuration used as the operation portion is not limited to the above-described configuration, and different operation portions may be used.

For example, by operating the response switch, the CPU 21A displays the self-optometry assistance screen (refer to FIG. 3) on the display portion 35B of the second information processing device 2B used by the examiner assisting the self-optometry.

As shown in FIG. 3, for example, in the present example, the optometry flow 60 of the procedure set for the self-optometry being carried out is displayed in the procedure display field 52. For example, the optometry flow 60 is a flow displaying a graphic in which a plurality of examination items are arranged in order of the examination procedure.

In the present example, the identification is displayed in which the procedure being performed at that time (examination item being performed) can be identified among the entire procedure. In the present example, as the identification, the examination being performed item is displayed on the procedure by an arrow 53 and a thick frame. With such a configuration, it is possible to easily grasp the examination currently being performed, and thus, it is possible to easily perform the study on the examination (for example, the necessity for redoing the examination, the necessity for changing the procedure of the self-optometry).

In the present example, by selecting a predetermined examination item from the displayed optometry flow 60 by the operator who is the examinee or the examiner, the CPU 21A receives the input of the selection instruction to select the predetermined examination items from the operator. Next, the CPU 21A performs the examination corresponding to the predetermined examination item based on the received input of the selection instruction. That is, by the operation on the optometry flow 60 by the operator, the examination desired by the operator can be performed.

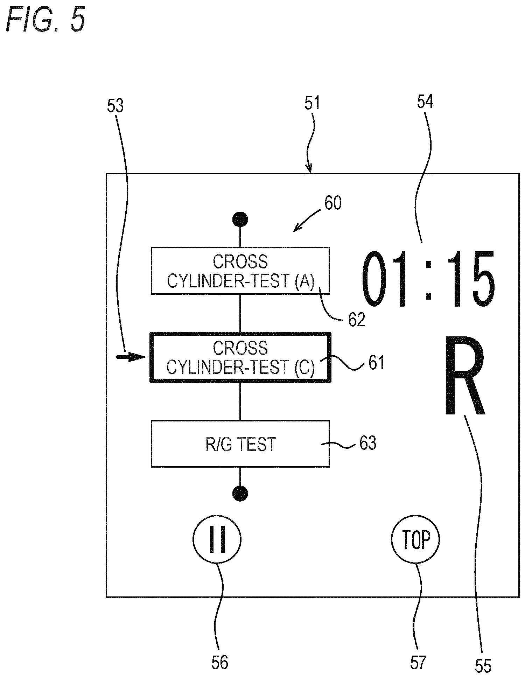

FIG. 5 is a diagram showing an example of a display screen displaying the optometry flow. The more detailed operation using the optometry flow will be described with reference to FIG. 5. In the present example, for example, the CPU 21A may display the examination item on the display portion 35B as the optometry flow 60 while restricting the examination item to a predetermined examination item including at least the examination item being performed. The optometry flow 60 in FIG. 5 displays the examination item in FIG. 3 (that is, the entire set examination item) while restricting the examination item to a predetermined examination item including the examination item being performed 61. With this configuration, for example, with regard to a plurality of examination items to be performed in the self-optometry, even if the number of optometry flows is large and it is difficult to check, it is easier to check the optometry flow in a certain range for the current examination item and other examination items, and thus, it becomes easier to grasp the current examination situation.

When the examination item is displayed, the displayed examination item being limited to the predetermined examination item, as the predetermined examination item to display on the display device as the optometry flow 60, the CPU 21A may display the examination item while restricting the examination item to the predetermined examination item further including at least one of the examination item that is performed after the examination item being performed and the examination item performed before the examination item being performed. For example, in FIG. 5, as the optometry flow 60, the examination item is restricted such that the examination item 62 performed before the examination item being performed 61 and the examination item 63 performed after the examination item being performed 61 are displayed on the display portion 35B. With this configuration, for example, since the examination item to be performed next (one item after) can be checked in addition to the examination item being performed, study for the optometry based on future examination (for example, the study with assuming the remaining time relating to the examination, the study whether the assistance is required in the next examination for the examinee who carries out the self-optometry, and the like) can be performed easily. In addition, for example, since the examination item performed before (one item before) can be checked in addition to the examination item being performed, study for the optometry based on the past examination situations (study for re-performing of the past optometry while taking the current examination situation into account, study of changing the procedure of the self-optometry based on the proceeding situation of past examination, and the like) can be performed easily. In addition, for example, since the examination items before and after the examination item being performed can be checked in addition to the examination item being performed, it is easier to grasp the examination situation of the self-optometry, and thus, more detailed study regarding the examination can be performed easily.

For example, when the examination item is displayed on the display portion 35B as the optometry flow 60, the displayed examination item being limited to a predetermined examination item, other examination items restricted to be displayed (examination items that are not displayed) may be displayed by the operation by the examiner. For example, the restricted examination item may be displayed on the display portion 35B by the examiner operating the operation portion 34B to perform a scroll operation (for example, a scroll operation in the vertical direction). The method of displaying other examination items restricted to be displayed is not limited to the configuration described above, and various methods may be used.

In the present example, when a call performing instruction is input, the limitation, in which the examination item is displayed, the displayed examination item being limited to a predetermined examination item, may be released. For example, when the examination item is displayed, the displayed examination item being limited to a predetermined examination item and when the call performing instruction is input, the CPU 21A may release the limitation on the examination item displayed as the optometry flow 60. That is, for example, the CPU 21A may release the restricted display of the optometry flow 60 in FIG. 5 and change it to the display of the optometry flow 60 (the entire set examination item) shown in FIG. 3. With this configuration, for example, when calling the examiner, since the entire optometry flow of a plurality of examination items performed in the self-optometry is displayed, the examiner can grasp the examination situation in the self-optometry more easily. As a result, it is easier to grasp the current situation of the self-optometry, and thus, it is possible to easily assist the self-optometry.

For example, in the present example, the operation on the optometry flow 60 may be restricted until the examiner is called for the self-optometry assistance. For example, the CPU 21A may restrict the input from the operation portion 34B until the input of call performing instruction is received. The input from the operation portion 34A may be restricted.

When the CPU 21A receives the input of call performing instruction to perform the call action for calling the examiner who is the operator, and may perform the call action based on the call performing instruction. In addition, for example, the CPU 21A may restrict the input of the selection instruction from the operation portion through which the examiner operates the subjective optometry device 1 until the call performing instruction is input, and when the call performing instruction is input, the CPU 21A may release the restriction on the input from the operation portion. With configuration, for example, since a selection operation from the operation portion used by the examiner is restricted, it is possible to prevent the examiner from touching the operation portion and interfering with the examination in the self-examination.

For example, when the call performing instruction is input, the operation by the operation portion 34B becomes valid by releasing the restriction on the input from the operation portion 34B. As a result, it becomes possible for the examiner to operate the operation portion 34B and to assist the examinee for the optometry. Therefore, for example, after selecting the examination item on the optometry flow 60, the examiner can input a verbal response of the examinee to the controller for the examiner, can change the target, and can change the calibration value.

For example, when restricting the input from the operation portion 34B, the input signal from the operation portion 34B may be blocked, or the response itself may be received but not involved in (ignore) the proceeding of the self-optometry. However, not all the operations by the operation portion 34B need to be restricted. For example, the input to an input unit to receive the input of a forced assistance for performing the forced assistance by the examiner or the input to an input unit for giving advice to the examinee by voice, letters, and like may not have to be restricted before the call performing instruction is input.

When the input of call performing instruction is received, the CPU 21A can quickly perform the assistance of the optometry by immediately releasing the restriction on the input from the operation portion 34B. However, timing of the CPU 21A to release the restriction on the input from the operation portion 34B is not limited to this. For example, the CPU 21A may release the restriction on the input from the operation portion 34B after a predetermined time elapses from the time when the input of call performing instruction is received. For example, when there is a response from the examiner saying that the optometry assistance can be performed, the CPU 21A may release the restriction on the input from the operation portion 34B.

For example, by selecting the desired examination item by the operation portion 34B while checking the optometry flow 60 displayed on the display screen of the display portion 35B, the examiner can easily select the examination item for carrying out the optometry assistance. As a result, the examiner can perform the examination of the selected examination item.

For example, when a predetermined examination item is selected, a setting is performed to start the examination for the selected examination item. For example, the CPU 21A performs at least one of the target setting by the target presentation portion 16 and the calibration value setting by the calibration optical system 11, associated with the selected examination item.

In FIG. 5, for example, when the operation portion 34B is operated by the examiner and the cross cylinder-test (C) is selected from the examination item of the examination item being performed 61, the CPU 21A starts the cross cylinder-test (C). For example, when starting the examination of the selected examination item, as the setting of a starting state when starting the examination, the CPU 21A may control at least one of the target presentation portion 16 and the calibration optical system 11 such the setting state becomes the examination state at the time of receiving the input of the selection instruction. In this case, for example, the examination state (for example, the calibration value and the target) at the time when the call performing instruction is input may be maintained and the CPU 21A may start the examination with the maintained examination state as the initial state.

For example, the examination starting state is not limited to the configuration described above, and various settings can be performed. For example, the CPU 21A may control at least one of the target presentation portion 16 and the calibration optical system 11 such that the setting state becomes the initial state (the state in which the initial value is set) of the examination item at the time of receiving the input of the selection instruction. In this case, for example, the target at the time of starting the examination of the examination item at the time of receiving the input of the selection instruction may be presented by the target presentation portion 16, or the calibration value at the time of starting the examination of the examination item at the time of receiving the input of the selection instruction may be set by the calibration optical system 11. As a result, for example, since the examination in the initial state of the examination at the time of receiving the input of the selection instruction can be performed, when the examination at the time of receiving the input of the selection instruction is inappropriately performed, the examination can be reset, the examination can be performed from the starting point of the examination, and as a result, the examination can be performed smoothly.

In addition, for example, the CPU 21A may control at least one of the target presentation portion 16 and the calibration optical system 11 such that the setting state is in a default state (for example, the state where the calibration amount is zero).

For example, after the cross cylinder-test (C) by the assistance of the examiner is completed, the self-optometry may be restarted. In this case, for example, after the examination of the cross cylinder-test (C) is completed, the operation portion 34B is operated by the examiner, and the examination item from which the self-optometry is restarted is selected in the optometry flow 60. That is, by the examiner, the self-optometry is restarted from the selected examination item.

For example, after the cross cylinder-test (C) is completed, when the operation portion 34B is operated by the examiner and the R / G test of the examination item 63 is selected, the CPU 21A starts the R / G test, and then, carries out the self-optometry according to the procedure. For example, when the operation portion 35B is operated by the examiner and the cross cylinder-test (A) of the examination item 62 is selected, the CPU 21A starts the cross cylinder-test (A), and then, carries out the self-optometry according to the procedure.

As described above, for example, the subjective optometry system in the present example includes a subjective optometry device, used for subjectively measuring the optical characteristics of the subject eye, having a calibration optical system that changes the optical characteristics of the target light flux presented to a subject eye and a target presentation portion that presents a target to the subject eye and a first information processing device connected to the subjective optometry device. For example, the first information processing device includes a self-optometry program that automatically proceeds with the optometry according to a response input by the examinee. For example, the self-optometry program performs at least a self-optometry proceeding step of controlling the calibration optical system and the target presentation portion to perform a plurality of examination items based on an automatically proceeded procedure of the self-optometry, and a self-optometry assistance step of performing an assistance action to assist the proceeding of the self-optometry when a problem occurs in the proceeding of the self-optometry. **In** addition, for example, the self-optometry assistance step includes a display control step of displaying an optometry flow in which a plurality of examination items are arranged in order of a procedure of the self-optometry on the display device, a selection receipt step of receiving an input of a selection instruction to select a predetermined examination item, from the operator, in the optometry flow displayed on the display device, and a selection performing step of performing the examination corresponding to the predetermined examination item based on the received input of the selection instruction. With such a configuration, for example, by checking the optometry flow displayed on the display device, it is possible to grasp the situation of the self-optometry such as the procedure and the situation of proceeding, and to select the examination item that the operator wants to assist. Therefore, for example, the operator can select the examination item to be assisted while appropriately grasping the situation of self-optometry such as the details of the procedure and the situation of proceeding by the displayed optometry flow, and can easily start the assistance-required examination. In addition, for example, at the time of self-optometry, even if the operator does not stay close to the examinee all the time and does not grasp the situation of the self-optometry, it is possible to easily start the examination that requires the assistance.

For example, in the selection performing step, the self-optometry may be restarted from the examination corresponding to the selected predetermined examination item based on the input of the selection instruction for the optometry flow. With such a configuration, it is possible to perform the instruction for the examination item to restart the self-optometry while checking the optometry flow displayed on the display device. Therefore, the operator can designate an examination process in which the self-optometry is restarted in a state where details of the procedure, the situation of proceeding, and the like is appropriately grasped by using the displayed optometry flow.

For example, the self-optometry assistance step may be performed according to an instruction input to a second information processing device, which is another information processing device connected to the first information processing device via a network. With this configuration, even if the examiner is in a remote location that is different from the place of examinee, the examiner can appropriately assist the self-optometry by examinee from the remote location by inputting the instruction to the second information processing device.

In the present example, at least one of a plurality of examination items and the procedure of the optometry flow 60 may be changed. In this case, for example, the operation portion 34B is operated by the examiner, at least one of the plurality of examination items and the procedure is changed. For example, the CPU 21A changes the display of the optometry flow 60 based on at least one of the plurality of examination items and the procedure changed by the examiner. For example, with this configuration, since the procedure can be changed appropriately, and the optometry flow corresponding to the change of the procedure can be displayed, it is possible to carry out the self-optometry considering intention of the examiner. In addition, for example, the self-optometry which is appropriate for the examinee becomes possible, and it is possible to proceed with the optometry smoothly.

In the above description, the call action for calling the examiner is performed as the call performing instruction, but any call action for calling the operator may be used. Examples of the operator include a staff member who does not perform the examination, and an attendant of the examinee in addition to the examiner who performs the subjective examination. Even when calls for the staff member and the attendant is performed, since a fact that the optometry assistance is required is notified, the examiner who can perform the subjective examination can be called via the staff member or the like. In addition, it can be expected that even the staff member may give a certain advice such as an adjustment of the positional relationship between the optometry device main body and the examinee.

## Claims

1. An optometry control program executed by a first information processing device (2A) in a subjective optometry system (100) that includes a subjective optometry (1) device which is used for subjectively measuring optical characteristics of a subject eye and which includes a calibration optical system (11) that changes optical characteristics of a target light flux presented to the subject eye and a target presentation portion (16) that presents a target to the subject eye and that includes a dedicated operation portion (34a) configured to input a response of the subjective optometry by an examinee, and the first information processing device (2A) connected to the subjective optometry device (1), the optometry control program comprising:
a self-optometry program configured to automatically proceed with an optometry according to the response input by the examinee via the operation portion (34a),
wherein the optometry control program comprises instructions which, when the self-optometry program is executed by a control portion (21A) of the first information processing device (2A), cause the first information processing device (2A) to perform:
a self-optometry proceeding step of controlling the calibration optical system (11) and the target presentation portion (16) to perform a plurality of examination items based on at least a procedure of a self-optometry that is automatically proceeded; and
a self-optometry assistance step of performing an assistance action to assist a proceeding of the self-optometry in a case where a problem occurs in the proceeding of the self-optometry, and
the self-optometry assistance step includes:
a display control step of displaying an optometry flow, on a display device (35A, 35B), in which the plurality of examination items are arranged in order of the procedure of the self-optometry;
a selection receipt step of receiving an operator's input of a selection instruction to select a predetermined examination item in the optometry flow displayed on the display device (35A, 35B); and
a selection performing step of performing an examination corresponding to the predetermined examination item based on the received input of the selection instruction.

2. The optometry control program according to claim 1,
wherein the self-optometry assistance step includes:
a call receipt step of receiving an input of a call performing instruction to perform a call action for calling an examiner who is the operator; and
a call performing step of performing the call action according to the call performing instruction received in the call receipt step, and
the control portion (21A) of the first information processing device (2A) restricts the input of the selection instruction from an operation portion (34B) through which the examiner operates the subjective optometry device (1) until the call performing instruction is input, and releases the restriction on the input from the operation portion in a case where the call performing instruction is input.

3. The optometry control program according to claim 1 or 2,
wherein, in the display control step, the examination item is displayed on the display device (35A, 35B) as the optometry flow, the displayed examination item being limited to a predetermined examination item including at least an examination item being performed.

4. The optometry control program according to claim 3,
wherein, in the display control step, the predetermined examination item is displayed on the display device (35A, 35B) as the optometry flow, the displayed predetermined examination item being limited to the predetermined examination item further including at least one of an examination item performed after the examination item being performed and an examination item performed before the examination item being performed.

5. The optometry control program according to claim 3 or 4,
wherein, in the display control step, when the examination item is displayed, the displayed examination item being limited to the predetermined examination item, the limitation on displaying the examination item as the optometry flow is released in a case where the call performing instruction is input.

6. The optometry control program according to any one of claims 1 to 5,
wherein, in the display control step, an identification by which at least an examination item being performed enables to be identified is displayed in the optometry flow.

7. The optometry control program according to any one of claims 1 to 6,
wherein, in the self-optometry assistance step, at least one of the procedure of the self-optometry and the plurality of examination items is variable according to an instruction input by the operator, and
in the display control step, the optometry flow is displayed on the display device (35A, 35B) while being changed based on at least one of the procedure of the self-optometry and the plurality of examination items changed according to the instruction input by the operator.

8. The optometry control program according to any one of claims 1 to 7,
wherein, in the selection performing step, the self-optometry is restarted from an examination corresponding to the selected predetermined examination item in the optometry flow based on the input of the selection instruction.

9. The optometry control program according to any one of claims 1 to 8,
wherein the self-optometry assistance step is performed according to an instruction input to a second information processing device (2B), which is another information processing device connected to the first information processing device (2A) via a network (5).

10. A subjective optometry system (100) comprising:
a subjective optometry device (1) which is used for subjectively measuring optical characteristics of a subject eye and which includes a calibration optical system (11) that changes optical characteristics of a target light flux presented to the subject eye and a target presentation portion (16) that presents a target to the subject eye and which includes a dedicated operation portion (34a) configured to input a response of the subjective optometry by an examinee; and
a first information processing device (2A) connected to the subjective optometry device (1),
wherein the first information processing device (2A) is configured to execute a self-optometry program that automatically proceeds with an optometry according to the response input by the examinee via the operation portion (34a),
the self-optometry program comprises instructions which, when the self-optometry program is executed by the first information processing device (2A), cause the first information processing device (2A) to perform:
a self-optometry proceeding step of controlling the calibration optical system (11) and the target presentation portion (16) to perform a plurality of examination items based on at least a procedure of a self-optometry that is automatically proceeded; and
a self-optometry assistance step of performing an assistance action to assist a proceeding of the self-optometry in a case where a problem occurs in the proceeding of the self-optometry, and
the self-optometry assistance step includes:
a display control step of displaying an optometry flow, on a display device (35A, 35B), in which the plurality of examination items are arranged in order of the procedure of the self-optometry;
a selection receipt step of receiving an operator's input of a selection instruction to select a predetermined examination item in the optometry flow displayed on the display device (35A, 35B); and
a selection performing step of performing an examination corresponding to the predetermined examination item based on the received input of the selection instruction.

## Patentansprüche

1. Optometrie-Steuerprogramm, das durch eine erste Informationsverarbeitungsvorrichtung (2A) in einem subjektiven Optometrie-System (100) ausgeführt wird, das eine subjektive Optometrie-Vorrichtung (1), die zum subjektiven Messen optischer Eigenschaften eines Auges eines Subjekts verwendet wird und die ein optisches Kalibrierungssystem (11), das optische Eigenschaften eines Ziellichtflusses ändert, der dem Auge des Subjekts präsentiert wird, und einen Zielpräsentationsabschnitt (16), der dem Auge des Subjekts ein Ziel präsentiert und der einen dedizierten Betriebsabschnitt (34a) beinhaltet, der konfiguriert ist, um eine Antwort der subjektiven Optometrie durch einen Probanden einzugeben, und die erste Informationsverarbeitungsvorrichtung (2A), die mit der subjektiven Optometrie-Vorrichtung (1) verbunden ist, beinhaltet, wobei das Optometrie-Steuerprogramm Folgendes umfasst:
ein Selbst-Optometrie-Programm, das konfiguriert ist, um automatisch mit einer Optometrie gemäß der Antwort fortzufahren, die durch den Probanden über den Betriebsabschnitt (34a) eingegeben wird,
wobei das Optometrie-Steuerprogramm Anweisungen umfasst, die, wenn das Selbst-Optometrie-Programm durch einen Steuerabschnitt (21A) der ersten Informationsverarbeitungsvorrichtung (2A) ausgeführt wird, die erste Informationsverarbeitungsvorrichtung (2A) veranlassen, Folgendes durchzuführen:
einen Selbst-Optometrie-Fortfahrschritt des Steuerns des optischen Kalibrierungssystems (11) und des Zielpräsentationsabschnitts (16), um eine Vielzahl von Untersuchungspunkten basierend auf mindestens einem Verfahren einer Selbst-Optometrie durchzuführen, die automatisch fortgefahren wird; und
einen Selbst-Optometrie-Unterstützungsschritt des Durchführens einer Unterstützungsaktion, um ein Fortfahren der Selbst-Optometrie in einem Fall zu unterstützen, in dem ein Problem beim Fortfahren der Selbst-Optometrie auftritt, und
der Selbst-Optometrie-Unterstützungsschritt Folgendes beinhaltet:
einen Anzeigesteuerschritt des Anzeigens eines Optometrie-Flusses auf einer Anzeigevorrichtung (35A, 35B), in der die Vielzahl von Untersuchungspunkten in der Reihenfolge des Verfahrens der Selbst-Optometrie angeordnet sind;
einen Auswahlempfangsschritt des Empfangens einer Eingabe eines Bedieners einer Auswahlanweisung zum Auswählen eines vorbestimmten Untersuchungspunkts in dem Optometrie-Fluss, der auf der Anzeigevorrichtung (35A, 35B) angezeigt wird; und
einen Auswahldurchführungsschritt des Durchführens einer Untersuchung, die dem vorbestimmten Untersuchungspunkt entspricht, basierend auf der empfangenen Eingabe der Auswahlanweisung.

2. Optometrie-Steuerprogramm nach Anspruch 1,
wobei der Selbst-Optometrie-Unterstützungsschritt Folgendes beinhaltet:
einen Anrufempfangsschritt des Empfangens einer Eingabe einer Anrufdurchführungsanweisung zum Durchführen einer Anrufaktion zum Anrufen eines Prüfers, der der Bediener ist; und
einen Anrufdurchführungsschritt des Durchführens der Anrufaktion gemäß der im Anrufempfangsschritt empfangenen Anrufdurchführungsanweisung, und
der Steuerabschnitt (21A) der ersten Informationsverarbeitungsvorrichtung (2A) die Eingabe der Auswahlanweisung von einem Betriebsabschnitt (34B), durch den der Prüfer die subjektive Optometrie-Vorrichtung (1) betreibt, beschränkt, bis die Anrufdurchführungsanweisung eingegeben wird, und die Beschränkung der Eingabe vom Betriebsabschnitt in einem Fall freigibt, in dem die Anrufdurchführungsanweisung eingegeben wird.

3. Optometrie-Steuerprogramm nach Anspruch 1 oder 2,
wobei im Anzeigesteuerschritt der Untersuchungspunkt auf der Anzeigevorrichtung (35A, 35B) als der Optometrie-Fluss angezeigt wird, wobei der angezeigte Untersuchungspunkt auf einen vorbestimmten Untersuchungspunkt beschränkt ist, der mindestens einen durchgeführten Untersuchungspunkt beinhaltet.

4. Optometrie-Steuerprogramm nach Anspruch 3,
wobei im Anzeigesteuerschritt der vorbestimmte Untersuchungspunkt auf der Anzeigevorrichtung (35A, 35B) als der Optometrie-Fluss angezeigt wird, wobei der angezeigte vorbestimmte Untersuchungspunkt auf den vorbestimmten Untersuchungspunkt beschränkt ist, der ferner mindestens einen von einem nach der Durchführung des Untersuchungspunkts durchgeführten Untersuchungspunkt und einem vor der Durchführung des Untersuchungspunkts durchgeführten Untersuchungspunkt beinhaltet.

5. Optometrie-Steuerprogramm nach Anspruch 3 oder 4,
wobei im Anzeigesteuerschritt, wenn der Untersuchungspunkt angezeigt wird, wobei der angezeigte Untersuchungspunkt auf den vorbestimmten Untersuchungspunkt beschränkt ist, die Beschränkung des Anzeigens des Untersuchungspunkts als der Optometrie-Fluss in einem Fall freigegeben wird, in dem die Anrufdurchführungsanweisung eingegeben wird.

6. Optometrie-Steuerprogramm nach einem der Ansprüche 1 bis 5,
wobei im Anzeigesteuerschritt eine Identifikation, durch die es ermöglicht wird, mindestens einen durchgeführten Untersuchungspunkt zu identifizieren, im Optometrie-Fluss angezeigt wird.

7. Optometrie-Steuerprogramm nach einem der Ansprüche 1 bis 6,
wobei im Selbst-Optometrie-Unterstützungsschritt mindestens eines des Verfahrens der Selbst-Optometrie und der Vielzahl von Untersuchungspunkten gemäß einer vom Bediener eingegebenen Anweisung variabel ist, und
im Anzeigesteuerschritt der Optometrie-Fluss auf der Anzeigevorrichtung (35A, 35B) angezeigt wird, während er basierend auf mindestens einem des Verfahrens der Selbst-Optometrie und der Vielzahl von Untersuchungspunkten geändert wird, die gemäß der vom Bediener eingegebenen Anweisung geändert werden.

8. Optometrie-Steuerprogramm nach einem der Ansprüche 1 bis 7,
wobei im Auswahldurchführungsschritt die Selbst-Optometrie von einer Untersuchung, die dem ausgewählten vorbestimmten Untersuchungspunkt im Optometrie-Fluss entspricht, basierend auf der Eingabe der Auswahlanweisung neu gestartet wird.

9. Optometrie-Steuerprogramm nach einem der Ansprüche 1 bis 8,
wobei der Selbst-Optometrie-Unterstützungsschritt gemäß einer Anweisungseingabe in eine zweite Informationsverarbeitungsvorrichtung (2B) durchgeführt wird, die eine andere Informationsverarbeitungsvorrichtung ist, die über ein Netzwerk (5) mit der ersten Informationsverarbeitungsvorrichtung (2A) verbunden ist.

10. Subjektives Optometrie-System (100), das Folgendes umfasst:
eine subjektive Optometrie-Vorrichtung (1), die zum subjektiven Messen optischer Eigenschaften eines Auges eines Subjekts verwendet wird und die ein optisches Kalibrierungssystem (11), das optische Eigenschaften eines Ziellichtflusses ändert, der dem Auge des Subjekts präsentiert wird, und einen Zielpräsentationsabschnitt (16), der dem Auge des Subjekts ein Ziel präsentiert und der einen dedizierten Betriebsabschnitt (34a) beinhaltet, der konfiguriert ist, um eine Antwort der subjektiven Optometrie durch einen Probanden einzugeben, beinhaltet; und
eine erste Informationsverarbeitungsvorrichtung (2A), die mit der subjektiven Optometrie-Vorrichtung (1) verbunden ist,
wobei die erste Informationsverarbeitungsvorrichtung (2A) konfiguriert ist, um ein Selbst-Optometrie-Programm auszuführen, das automatisch mit einer Optometrie gemäß der Antwort fortfährt, die durch den Probanden über den Betriebsabschnitt (34a) eingegeben wird,
wobei das Selbst-Optometrie-Programm Anweisungen umfasst, die, wenn das Selbst-Optometrie-Programm durch die erste Informationsverarbeitungsvorrichtung (2A) ausgeführt wird, die erste Informationsverarbeitungsvorrichtung (2A) veranlassen, Folgendes durchzuführen:
einen Selbst-Optometrie-Fortfahrschritt des Steuerns des optischen Kalibrierungssystems (11) und des Zielpräsentationsabschnitts (16), um eine Vielzahl von Untersuchungspunkten basierend auf mindestens einem Verfahren einer Selbst-Optometrie durchzuführen, die automatisch fortgefahren wird; und
einen Selbst-Optometrie-Unterstützungsschritt des Durchführens einer Unterstützungsaktion, um ein Fortfahren der Selbst-Optometrie in einem Fall zu unterstützen, in dem ein Problem beim Fortfahren der Selbst-Optometrie auftritt, und
der Selbst-Optometrie-Unterstützungsschritt Folgendes beinhaltet:
einen Anzeigesteuerschritt des Anzeigens eines Optometrie-Flusses auf einer Anzeigevorrichtung (35A, 35B), in der die Vielzahl von Untersuchungspunkten in der Reihenfolge des Verfahrens der Selbst-Optometrie angeordnet sind;
einen Auswahlempfangsschritt des Empfangens einer Eingabe eines Bedieners einer Auswahlanweisung zum Auswählen eines vorbestimmten Untersuchungspunkts im Optometrie-Fluss, der auf der Anzeigevorrichtung (35A, 35B) angezeigt wird; und
einen Auswahldurchführungsschritt des Durchführens einer Untersuchung, die dem vorbestimmten Untersuchungspunkt entspricht, basierend auf der empfangenen Eingabe der Auswahlanweisung.

## Revendications

1. Programme de commande d'optométrie exécuté par un premier dispositif de traitement d'informations (2A) dans un système d'optométrie subjective (100) qui comprend un dispositif d'optométrie subjective (1) qui est utilisé pour mesurer subjectivement des caractéristiques optiques d'un oeil d'un sujet et qui comprend un système optique d'étalonnage (11) qui change des caractéristiques optiques d'un flux lumineux cible présenté à l'oeil du sujet et une partie de présentation de cible (16) qui présente une cible à l'oeil du sujet et qui comprend une partie d'opération dédiée (34a) configurée pour entrer une réponse de l'optométrie subjective par un examiné, et le premier dispositif de traitement d'informations (2A) connecté au dispositif d'optométrie subjective (1), le programme de commande d'optométrie comprenant :
un programme d'auto-optométrie configuré pour procéder automatiquement à une optométrie selon la réponse entrée par l'examiné via la partie d'opération (34a),
dans lequel le programme de commande d'optométrie comprend des instructions qui, lorsque le programme d'auto-optométrie est exécuté par une partie de commande (21A) du premier dispositif de traitement d'informations (2A), amènent le premier dispositif de traitement d'informations (2A) à effectuer :
une étape de procédé d'auto-optométrie consistant à commander le système optique d'étalonnage (11) et la partie de présentation de cible (16) pour effectuer une pluralité d'éléments d'examen sur la base d'au moins une procédure d'une auto-optométrie qui est procédée automatiquement ; et
une étape d'assistance d'auto-optométrie consistant à effectuer une action d'assistance pour assister un procédé de l'auto-optométrie dans un cas où un problème se produit dans le procédé de l'auto-optométrie, et
l'étape d'assistance d'auto-optométrie comprend :
une étape de commande d'affichage consistant à afficher un flux d'optométrie, sur un dispositif d'affichage (35A, 35B), dans lequel la pluralité d'éléments d'examen sont agencés dans l'ordre de la procédure de l'auto-optométrie ;
une étape de réception de sélection consistant à recevoir une entrée d'opérateur d'une instruction de sélection pour sélectionner un élément d'examen prédéterminé dans le flux d'optométrie affiché sur le dispositif d'affichage (35A, 35B) ; et
une étape d'exécution de sélection consistant à effectuer un examen correspondant à l'élément d'examen prédéterminé sur la base de l'entrée reçue de l'instruction de sélection.

2. Programme de commande d'optométrie selon la revendication 1,
dans lequel l'étape d'assistance d'auto-optométrie comprend :
une étape de réception d'appel consistant à recevoir une entrée d'une instruction d'exécution d'appel pour effectuer une action d'appel pour appeler un examinateur qui est l'opérateur ; et
une étape d'exécution d'appel consistant à effectuer l'action d'appel selon l'instruction d'exécution d'appel reçue à l'étape de réception d'appel, et
la partie de commande (21A) du premier dispositif de traitement d'informations (2A) restreint l'entrée de l'instruction de sélection à partir d'une partie d'opération (34B) par le biais de laquelle l'examinateur actionne le dispositif d'optométrie subjective (1) jusqu'à ce que l'instruction d'exécution d'appel soit entrée, et libère la restriction sur l'entrée à partir de la partie d'opération dans un cas où l'instruction d'exécution d'appel est entrée.

3. Programme de commande d'optométrie selon la revendication 1 ou 2,
dans lequel, à l'étape de commande d'affichage, l'élément d'examen est affiché sur le dispositif d'affichage (35A, 35B) en tant que flux d'optométrie, l'élément d'examen affiché étant limité à un élément d'examen prédéterminé comprenant au moins un élément d'examen qui est effectué.

4. Programme de commande d'optométrie selon la revendication 3,
dans lequel, à l'étape de commande d'affichage, l'élément d'examen prédéterminé est affiché sur le dispositif d'affichage (35A, 35B) en tant que flux d'optométrie, l'élément d'examen prédéterminé affiché étant limité à l'élément d'examen prédéterminé comprenant en outre au moins l'un d'un élément d'examen effectué après l'élément d'examen qui est effectué et d'un élément d'examen effectué avant l'élément d'examen qui est effectué.

5. Programme de commande d'optométrie selon la revendication 3 ou 4,
dans lequel, à l'étape de commande d'affichage, lorsque l'élément d'examen est affiché, l'élément d'examen affiché étant limité à l'élément d'examen prédéterminé, la limitation sur l'affichage de l'élément d'examen en tant que flux d'optométrie est libérée dans un cas où l'instruction d'exécution d'appel est entrée.

6. Programme de commande d'optométrie selon l'une quelconque des revendications 1 à 5,
dans lequel, à l'étape de commande d'affichage, une identification par laquelle au moins un élément d'examen qui est effectué permet d'être identifié est affichée dans le flux d'optométrie.

7. Programme de commande d'optométrie selon l'une quelconque des revendications 1 à 6,
dans lequel, à l'étape d'assistance d'auto-optométrie, au moins l'un de la procédure de l'auto-optométrie et de la pluralité d'éléments d'examen est variable selon une instruction entrée par l'opérateur, et
à l'étape de commande d'affichage, le flux d'optométrie est affiché sur le dispositif d'affichage (35A, 35B) tout en étant modifié sur la base d'au moins l'un de la procédure de l'auto-optométrie et de la pluralité d'éléments d'examen modifiés selon l'instruction entrée par l'opérateur.

8. Programme de commande d'optométrie selon l'une quelconque des revendications 1 à 7,
dans lequel, à l'étape d'exécution de sélection, l'auto-optométrie est redémarrée à partir d'un examen correspondant à l'élément d'examen prédéterminé sélectionné dans le flux d'optométrie sur la base de l'entrée de l'instruction de sélection.

9. Programme de commande d'optométrie selon l'une quelconque des revendications 1 à 8,
dans lequel l'étape d'assistance d'auto-optométrie est effectuée selon une entrée d'instruction dans un second dispositif de traitement d'informations (2B), qui est un autre dispositif de traitement d'informations connecté au premier dispositif de traitement d'informations (2A) par le biais d'un réseau (5).

10. Système d'optométrie subjective (100) comprenant :
un dispositif d'optométrie subjective (1) qui est utilisé pour mesurer subjectivement des caractéristiques optiques d'un oeil d'un sujet et qui comprend un système optique d'étalonnage (11) qui change des caractéristiques optiques d'un flux lumineux cible présenté à l'oeil du sujet et une partie de présentation de cible (16) qui présente une cible à l'oeil du sujet et qui comprend une partie d'opération dédiée (34a) configurée pour entrer une réponse de l'optométrie subjective par un examiné ; et
un premier dispositif de traitement d'informations (2A) connecté au dispositif d'optométrie subjective (1),
dans lequel le premier dispositif de traitement d'informations (2A) est configuré pour exécuter un programme d'auto-optométrie qui procède automatiquement à une optométrie selon la réponse entrée par l'examiné via la partie d'opération (34a),
le programme d'auto-optométrie comprend des instructions qui, lorsque le programme d'auto-optométrie est exécuté par le premier dispositif de traitement d'informations (2A), amènent le premier dispositif de traitement d'informations (2A) à effectuer :
une étape de procédé d'auto-optométrie consistant à commander le système optique d'étalonnage (11) et la partie de présentation de cible (16) pour effectuer une pluralité d'éléments d'examen sur la base d'au moins une procédure d'une auto-optométrie qui est procédée automatiquement ; et
une étape d'assistance d'auto-optométrie consistant à effectuer une action d'assistance pour assister un procédé de l'auto-optométrie dans un cas où un problème se produit dans le procédé de l'auto-optométrie, et
l'étape d'assistance d'auto-optométrie comprend :
une étape de commande d'affichage consistant à afficher un flux d'optométrie, sur un dispositif d'affichage (35A, 35B), dans lequel la pluralité d'éléments d'examen sont agencés dans l'ordre de la procédure de l'auto-optométrie ;
une étape de réception de sélection consistant à recevoir une entrée d'opérateur d'une instruction de sélection pour sélectionner un élément d'examen prédéterminé dans le flux d'optométrie affiché sur le dispositif d'affichage (35A, 35B) ; et
une étape d'exécution de sélection consistant à effectuer un examen correspondant à l'élément d'examen prédéterminé sur la base de l'entrée reçue de l'instruction de sélection.
